# EUROPEAN PATENT APPLICATION

(11) **EP 4 656 174 A1**
(43) Date of publication of application: **03.12.2025**
(21) Application number: 24178240.8
(22) Date of filing: 27.05.2024
(51) Int. Cl.: A61K 6/00, A61K 8/00, A61K 9/00, A61K 35/32, A61K 35/33, A61K 38/17, A61L 27/22, A61L 27/38, A61P 1/02, A61P 43/00

(54) **INJECTABLE COMPOSITIONS COMPRISING ENAMEL MATRIX DERIVATIVE (EMD) PROTEINS AND HUMAN SOFT TISSUE CELLS**

(71) Applicant: Institut Straumann AG, 4052 Basel (CH)
(72) Inventor: DARD, Michel, 4052 Basel (CH); WÄNGNERUD, Per, 214 32 Malmö (SE)
(74) Representative: Aera A/S

(57) **Abstract**

The present invention relates to a pharmaceutical, dental and/or cosmetic composition comprising a cell culture population of human fibroblasts (hPFB) and/or human periodontal ligament cells (hPDL), and purified and/or isolated enamel matrix derivative (EMD) proteins, which have a molecular weight between 1-160kDa and its use in treating patients suffering from a gingival deficiency and/or disorder, in particular such patients suffering from a gingival deficiency and/or disorder selected from the group consisting of gingival thin biotype, gingival recession and enlargement and gingival papilla regeneration. Preferably, the composition of the invention is administered via injection into the soft tissue in the oral cavity and/or the craniomaxillofacial complex (CMF) of the patient.

## Description

### Technical field

The present invention relates to the field of healing, restoration and/or promotion of soft tissue in the craniomaxillofacial complex (CMF), such as treating a gingival and/or oral mucosa wound, deficiency and/or disorder, and problems associated with the same.

A pharmaceutical, dental and/or cosmetic composition is herein disclosed for use in promoting growth and/or healing of soft tissue in the craniomaxillofacial complex (CMF), comprising isolated enamel matrix derivative (EMD) proteins, either as lyophilized powder, and/or within a suitable pharmaceutical carrier together with a cell culture population of human fibroblasts (hPFB) and/or human periodontal ligament cells (hPDL)

### Background

Gingival wound healing after dental treatment comprises a series of sequential responses that allow the closure of breaches in the masticatory mucosa. This process is of critical importance to prevent the invasion of microbes or other agents into tissues in the craniomaxillofacial complex (CMF), avoiding the establishment of a chronic infection. It also plays an important role during cell and tissue reaction to long-term injury, as may occur during inflammatory responses and cancer in the CMF.

The normal response to injury in soft tissue involves 3 overlapping stages: inflammation, new tissue formation, and remodelling (Smith P.C., et al, 2015, Journal of Dental Research).

Maxillofacial and/or oral surgeons', dental implantologists' and periodontists' main challenges come from having a limited supply of well-suited local tissues that are needed to approximate lost bone, muscle, nerves, skin, and mucosa. Local regional and distant flap harvests have been utilized in an attempt to increase the complexity of available tissues, with improved outcomes. Even though the availability of tissues allows the closure of a defect and obturation of dead space, the function and aesthetic goals are not yet ideally met.

Craniofacial and/or oral soft tissue reconstruction may be required following dental treatment, infection, trauma, tumour resection, and to repair congenital deformities. Successfully reconstructed tissue through soft tissue engineering protocols would help surgeons to restore the form and function of the lost tissue in its originality.

After tissue injury, distinct biological pathways immediately become activated and are synchronized to prevent infection and to restore the damaged tissues. The cells recruited during wound healing include components of the immune system (neutrophils, monocytes, lymphocytes, and dendritic cells), endothelial cells, keratinocytes, and fibroblasts. These cells undergo massive fluctuations in gene expression, commanding important changes in cell proliferation, differentiation, and migration. The extra-cellular matrix (ECM) also plays an important role in wound healing. During tissue repair, cells must secrete and organize components of the ECM such as collagens, fibronectin, proteoglycans, and matricellular proteins, among others. This response is critically important to fill the damaged tissues with new matrix components to allow the migration of cells and to remodel tissues after injury. Moreover, the ECM provides a physical support by assisting as a cell framework.

Gingival tissue biotype is a critical factor that determines the result of gingival wound healing, such as after dental treatment (e.g.., periodontology, implant dentistry). The initial gingival thickness is particularly significant as it may predict the outcome of root (natural, metallic or ceramic) coverage procedures and restorative treatments. There are mainly two different types of gingival biotypes recognized in the field of dentistry, the thin gingival biotype and the thick gingival biotype. Different gingival biotypes respond differently to inflammation, restorative, trauma and parafunctional habits. These traumatic events result in various types of periodontal and/or periimplant defects, which respond differently to different treatments.

Gingival biotype has a significant impact on the outcome of restorative and regenerative therapy. The disparity in treatment outcome is possibly because of the difference in tissue response to trauma. Hence in clinical practice identification of the periodontal biotype is significant. Gingival thickness can be assessed by various invasive and non-invasive methods. Thick and thin tissues often respond differently to inflammation and trauma (Abraham S. at al, 2013 The Saudi Journal for Dental Research).

The gingival thickness affects the treatment outcome in particular because of the difference in the amount of blood supply to the underlying bone and susceptibility to resorption. Gingival or periodontal and/or periimplant diseases are also more likely to occur in patients with a thin biotype and the remodelling process, after tooth extraction results in more dramatic alveolar resorption in the apical and lingual directions. An atraumatic extraction and preservation of the alveolar plates are essential, if the site is to be used for implant placement. When compromise of the alveolar plate is expected, it is then necessary to utilize ridge augmentation protocols.

As of today, periodontal and/or periimplant dentistry surgical techniques have been tried to improve the tissue quality and treatment outcome. E.g., soft tissue grafting in areas of thin biotypes can enhance the quality of the gingival tissue. To convert a thin soft tissue to a thick biotype, it is recommended to perform subepithelial connective tissue grafting. Other soft tissue augmentation procedures include modified roll technique and use of acellular dermal matrix. Finally, oral physiotherapy can improve tissue keratinization.

Still, since all surgical procedures in the oral cavity introduce a high risk of secondary infection and inflammatory complications, there is a long-felt need for a less invasive method to improve the tissue quality and treatment outcome of the soft tissues in the craniomaxillofacial complex.

### Summary of the invention

The present invention, for the first time, discloses a means and a method to enable a minimally-invasive treatment and/or surgery to improve the tissue quality and treatment outcome of the soft tissues in the craniomaxillofacial complex (CMF), in particular for patients with a thin gingival biotype.

The present invention relates to a pharmaceutical, dental and/or cosmetic composition comprising purified and/or isolated enamel matrix derivative (EMD) proteins, which have a molecular weight between 1-160kDa supplemented with a cell culture population of human fibroblasts (hPFB) and/or human periodontal ligament cells (hPDL), and its use for treating patients suffering from a gingival deficiency and/or disorder, in particular such patients suffering from a gingival deficiency and/or disorder selected from the group consisting of gingival thin biotype, gingival recession and enlargement and gingival papilla regeneration. Preferably, the composition of the invention is administered via injection, such as, but not limited to, by hydrodissection, into the soft tissue in the oral cavity and/or the craniomaxillofacial complex (CMF) of the patient.

The current invention for the first time discloses a pharmaceutical, dental and/or cosmetic composition comprising purified and/or isolated enamel matrix derivative (EMD) proteins, optionally in combination with other Extra Cellular Matrix proteins (collagen, fibronectin, proteoglycans, etc), which have a molecular weight between 1-160kDa, supplemented with a cell culture population of human fibroblasts (hPFB) and/or human periodontal ligament cells (hPDL)

In a currently preferred embodiment, the cells are oral human fibroblasts (hPFB) and the proteins are isolated porcine Enamel Matrix Derivative (EMD) proteins. Typically, the EMD proteins comprise amelogenins, enamelin, tuft protein, proteases and albumin and typically, at least 80% of the EMD proteins are amelogenins.

In a further aspect, the cells are oral human fibroblasts (hPFB) and the proteins are isolated porcine Enamel Matrix Derivative (EMD) proteins, in combination with other Extra Cellular Matrix proteins (collagen, fibronectin, proteoglycans, etc)

In embodiments, the cells and/or the EMD proteins have been lyophilized before being added to the composition.

A pharmaceutic dental and/or cosmetic composition according to the current invention can be formulated as a lyophilized powder or as a gel, such as a hydrogel.

A pharmaceutic dental and/or cosmetic composition according to the current invention can further comprise a suitable pharmaceutical carrier, which typically has a neutral pH, such as between 7-7.75, such a pH of about 7.5. When the composition is a solution, a liquid and/or an aqueous fluid, or in the form of an injectable gel, such as hydrogels, the carrier can be selected from the group consisting of sodium carboxymethyl cellulose, hyaluronic acid (HYA), poloxamers and/or Propylene Glycol Alginate (PGA).

A pharmaceutic dental and/or cosmetic composition according to the current invention can further comprise one or more growth factor(s).

One aspect of the current invention relates to a pharmaceutic dental and/or cosmetic composition according to the current invention, for use in medicine, such as, but not limited to, for use in minimally-invasive surgery. Typically, a pharmaceutic dental and/or cosmetic composition according to the current invention is administered by injection, such as assisted by hydrodissection.

In particular, a pharmaceutic dental and/or cosmetic composition according to the current invention is intended for use in inducing and/or promoting novel formation of soft tissue in the oral cavity and/or the craniomaxillofacial complex (CMF), for use in promoting growth and/or healing of soft tissue in the oral cavity and/or the craniomaxillofacial complex (CMF), for use in promoting reinforcement and/or thickening of gingiva in patients with a thin gingiva biotype, for use in treating a gingival deficiency and/or disorder selected from the group consisting of gingival thin biotype, gingival recession and enlargement and gingival papilla regeneration, for use in promoting gingival sealing around dental implants (periimplantitis prevention and treatment) and/or alveolar ridge preservation, and for use in extraction socket management, such as for treating intrabony pockets and/or promoting alveolar ridge regeneration (periodontology).

In one aspect, a pharmaceutic dental and/or cosmetic composition according to the current invention can be used in combination with an implantable 3D scaffold, such as a 3D scaffold-sponge. Such a combination is particularly useful in treating a deficiency and/or disorder selected from the group consisting of gingival recession and/or enlargement, alveolar ridge augmentation (implant dentistry), and intrabony pockets and/or alveolar ridge regeneration (periodontology), and/or for promoting alveolar ridge preservation and/or extraction socket management.

The current invention further relates to a kit comprising purified and/or isolated enamel matrix derivative (EMD) proteins, which have a molecular weight between 1-160kDa supplemented with a cell culture population of human fibroblasts (hPFB) and/or human periodontal ligament cells (hPDL) optionally further comprising a suitable pharmaceutical carrier. In such a kit, the cells are preferably oral human fibroblasts (hPFB) and the ExtraCellular Matrix proteins are preferably EMD isolated porcine Enamel Matrix Derivative (EMD) proteins, optionally in combination with collagen, fibronectin and/or proteoglycans. The carrier typically has a neutral pH, such as between 7-7.75.

A kit according to the current invention can be used for producing and/or manufacturing a pharmaceutic dental and/or cosmetic composition in the form of a powder, an injectable gel or injectable solution, or a liquid and/or an aqueous fluid, for use according to the current invention.

Finally, the current invention in one aspect relates to a method of treating a patient suffering from a gingival deficiency and/or disorder selected from the group consisting of gingival thin biotype, gingival recession and enlargement and gingival papilla regeneration, wherein said patient is injected with a composition according to the current invention.

### Definitions

### Biocompatible

The term "biocompatible" as used herein refers to causing no clinically relevant tissue irritation, injury, toxic reaction, or immunological reaction to living tissue.

### Bio- markers

The term "bio- markers" (or "biosignatures") as used herein refers to peptides, proteins, nucleic acids, antibodies, genes, metabolites, or any other substances used as indicators of a biologic state. It is a characteristic that is measured objectively and evaluated as a cellular or molecular indicator of normal biologic processes, pathogenic processes, or pharmacologic responses to a therapeutic intervention.

### Cell

The term "cell" is used herein to refer to the structural and functional unit of living organisms and is the smallest unit of an organism classified as living.

### CMF

The abbreviation "CMF" herein stands for craniomaxillofacial complex, i.e., the anatomical area of the mouth, jaws, face, skull, as well as associated structures.

### Compatible

The term "compatible" as used herein means that components of a composition are capable of being combined with each other in a manner such that there is no interaction that would substantially reduce the efficacy of the composition under ordinary use conditions.

### Component

The term "component" as used herein refers to a constituent part, element, or ingredient.

### Condition

The term "condition" as used herein refers to a variety of health states and is meant to include disorders or diseases caused by any underlying mechanism or disorder, injury, and the promotion of healthy tissues and organs.

### Dental device

A dental device according to the current invention can be a dental implant system. A dental implant system can in the current context comprise a dental implant, a crown and/or an abutment.

### Differentiation

The term "differentiation" as used herein refers to the process of development with an increase in the level of organization or complexity of a cell or tissue, accompanied with a more specialized function.

### Disease

The terms "disease" and "disorder" as used herein refer to an impairment of health or a condition of abnormal functioning.

### Endogenous

The term "endogenous" as used herein refers to that which is naturally occurring, incorporated within, housed within, adherent to, attached to, or resident in.
**ECM**
Extra Cellular Matrix
**EMD**
Enamel matrix derivative
**hPFB**
human fibroblasts
**hPDL**
human periodontal ligament cells
**HOrF**
Human Oral Fibroblasts

### Hydrodissection

Hydrodissection is the use of a directed jet of water to surgically separate tissues. It is generally used to develop tissue planes or divide soft tissues with less trauma than dissection using a cutting instrument.

### Mucosa

The term "mucosa" as used herein refers to a mucous tissue lining various tubular structures consisting of epithelium, lamina propria, and, in the digestive tract, a layer of 45 smooth muscle. The term "mucosal graft" as used herein refers to a graft of mucus membrane.

### Patient

The term "patient" as used herein refers to any mammal. Examples of mammals are humans, farm animals and domestic animals.

### Peptide

The term "peptide" is used herein to refer to two or more amino acids joined by a peptide bond.

### Periodontal phenotype

The term "periodontal phenotype" herein means periodontal biotype.

### Periodontitis

Inflammation of the tissue around the teeth, often causing shrinkage of the gums and loosening of the teeth

### Peri-implantitis

"Peri-implantitis" or "periimplantitis" is a dental term used to describe the destructive inflammatory process affecting the soft and hard tissues surrounding dental implants. Compared to mucositis, the definition of peri-implantitis includes bone loss. Among others, smoking, accumulation of bacterial biofilms (plaque), oral hygiene and periodontal status are influential factors. In the present context, the term "periodontal diseases" encompasses peri-implant infections, such as periimplantitis.

### Purification

The term "purification" as used herein refers to a process of isolating or freeing from foreign, extraneous, or objectionable elements so that the proportion of the desired substance or material is increased (enriched) relative to the starting material. The term is in the current context used interchangeably with "isolation"

It must be noted that as used herein and in the appended claims, the singular forms "a", "an", and "the" include plural references unless the context clearly dictates otherwise.

As used herein, the term "comprising" means the presence of the stated features, integers, steps, or components as referred to in the claims, but that it does not preclude the presence or addition of one or more other features, integers, steps, components or groups thereof. The term "comprising" is intended to include embodiments encompassed by the terms "consisting essentially of' and "consisting of'. Similarly, the term "consisting essentially of' is intended to include embodiments encompassed by the term "consisting of'

As used herein, the term "about" modifying the quantity of an ingredient or reactant employed refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term "about" also encompasses amounts that differ due to different equilibrium conditions for a composition resulting from a particular initial mixture. Whether or not modified by the term "about", the claims include equivalents to the quantities.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding both of those included limits are also included in the invention.

It is to be understood that the terminology used herein is for the purpose of describing particular embodiments only and is not intended to limit the scope of the present invention which will be limited only by the appended claims.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, exemplary methods and materials have been described. All publications mentioned herein are incorporated to disclose and described the methods and/or materials in connection with which the publications are cited.

### Figure legends

**Figure 1****:** By spinning samples of EMD at 1700G for 5 minutes it was indeed observed that part of the EMD may sediment into a pellet, leaving a somewhat less cloudy supernatant fraction.
**Figure 2****:** By loading unfractionated EMD and EMD supernatant into a standardized counting chamber it was possible to better visualize the microscopic characteristics of EMD.
**Figure 3****:** Assessment of cell numbers in wells analyzed at 24hr, 48hr, and 72hr indicated nominal morphology and cell growth as compared to the control setting.
**Figure 4****:** Assessment of cell numbers in wells analyzed at 24hr, 48hr, and 72hr indicated temporary decrease in available cell numbers after EMD exposure, but with the cell numbers having normalized at the 72hr time-point.
**Figure 5****:** Assessment of cell numbers in wells analyzed at 24hr, 48hr, and 72hr indicated a clear disadvantage to cell growth in the wells that contained media with 20% EMD supernatant.
**Figure 6****:** Assessment of cell numbers in wells analyzed at 24hr, 48hr, and 72hr indicated a significant disadvantage to cell growth in the wells that contained media with 20% EMD.
**Figure 7****:** Observation indicating the presence of surviving cells.
**Figure 8****:** Using Trypan Blue staining on a suspension of cells in EMD (post centrifugation) it was confirmed that HOrF cells exposed to EMD retain viability.

### Detailed description of invention

The present invention relates to a pharmaceutical, dental and/or cosmetic composition comprising a cell culture population of human fibroblasts (hPFB) and/or human periodontal ligament cells (hPDL) and purified and/or isolated enamel matrix derivative (EMD) proteins, either as lyophilized powder, or within a suitable pharmaceutical carrier and its use in treating patients suffering from a gingival deficiency and/or disorder, in particular such patients suffering from a gingival deficiency and/or disorder selected from the group consisting of gingival thin biotype, gingival recession and enlargement and gingival papilla regeneration. The herein for the first-time disclosed composition is also intended to be use in soft tissue reconstruction. Preferably, the composition of the invention is administered via injection, such by hydrodissection, into the soft tissue in the oral cavity and/or the craniomaxillofacial complex (CMF) of the patient, such as into the gingiva and/or the oral mucosa.

As is demonstrated in the experimental section, the application of a composition according to the current invention remarkably improves the cell growth of the gingiva cells and other fibroblasts in the CMF.

Importantly, the administration of the composition of the current invention can be done by a minimally invasive surgery, such as, by injection directly into the soft tissue in the CMF, e.g., assisted by hydrodissection. Thereby, the tissue to which the composition is administered will suffer a minimal administration-trauma, which reduces the risk for infection and/or inflammation due to the administration procedure.

### Periodontium

The periodontium is the assembly of specialized tissues that both surround and support the teeth, maintaining them in the maxillary and mandibular bones. The tissues of the periodontium combine to form an active, dynamic group of tissues. The alveolar bone is surrounded for the most part by the subepithelial connective tissue of the gingiva, which in turn is covered by the various characteristic gingival epithelia. The cementum overlaying the tooth root is attached to the adjacent cortical surface of the alveolar bone by the alveolar crest, horizontal and oblique fibres of the periodontal ligament.

It consists of four principal components, namely:
1. Gingiva
2. Periodontal ligament (PDL)
3. Cementum
4. Alveolar bone proper

Each of these components is distinct in location, architecture, and biochemical properties, which adapt during the life of the structure. For example, as teeth respond to forces or migrate medially, bone resorbs on the pressure side and is added on the tension side. Cementum similarly adapts to wear on the occlusal surfaces of the teeth by apical deposition. The periodontal ligament in itself is an area of high turnover that allows the tooth not only to be suspended in the alveolar bone but also to respond to the forces. Thus, although seemingly static and having functions of their own, all of these components function as a single unit.

The attachment of the tooth to the surrounding and supporting structures (bone) is accomplished through the cementum of the tooth, periodontal ligaments and the alveolar bone. The junctional epithelium is located at the base of the sulcus. It is adjacent to the tooth and is that part of the gingiva that attaches the connective tissue to the tooth. The root of the tooth (cementum) is attached to the underlying bone by a series of periodontal fibres that make up the periodontal ligament and allow for minor movement of the tooth in the socket without damage to the tooth or the underlying structures. These fibres are classified apical, oblique, horizontal, alveolar crest and interradicular fibres.

### Soft tissue

The currently described pharmaceutic dental and/or cosmetic composition is administered onto and/or into the soft tissue in the oral cavity and/or the craniomaxillofacial complex (CMF).

The term "soft tissue" as used herein refers to tissues that connect, support, or surround other structures and organs of the body. Soft tissue includes muscles, tendons, ligaments, fascia, nerves, fibrous tissues, fat, blood vessels, and synovial membranes. It gives shape to and supports the body, protects other body tissues and structures, and holds them together.

### Soft tissue in the oral cavity and/or the craniomaxillofacial complex (CMF)

The predominant soft tissue types in the CMF are the gingiva and the periodontal ligament.

A pharmaceutic dental and/or cosmetic composition according to the current invention can be used for inducing and/or promoting novel formation of soft tissue in the oral cavity and/or the craniomaxillofacial complex (CMF), such as of gingiva and/or the periodontal ligament. In one embodiment, it is used for promoting growth and/or healing of soft tissue in the oral cavity and/or the craniomaxillofacial complex (CMF), such as of the gingiva and/or the periodontal ligament.

### Gingiva

The makeup of the gingival tissue varies according to its location and function. There are two types of gingivae and several important anatomic regions.
1. Alveolar mucosa - The area of tissue beyond the mucogingival junction. It seems less firmly attached and redder than the attached gingiva. It is non-keratinized and provides a softer and more flexible area for the movement of the cheeks and lips.
2. Attached gingiva - This tissue is adjacent to the free gingiva and is keratinized and firmly attached to the bone structure. It can range from 3-12 mm in height.
3. Free gingiva - This tissue is not attached and forms a collar around the tooth. The trough around the tooth is called the sulcus and its depth is normally 1-3 mm. It is lined with sulcular epithelium and attached to the tooth at its base by the epithelial attachment. The sulcular epithelium is that epithelium which lines the gingival sulcus. It is apically bounded by the junctional epithelium and meets the epithelium of the oral cavity at the height of the free gingival margin.
4. Gingival margin - The border region of the gingiva that touches the tooth.
5. Junctional epithelium - The part of the gingiva that attaches the connective tissue to the tooth. It is located at the base of the sulcus.
6. Interdental papillae - The region of gingival tissue that fills the space between adjacent teeth. In a healthy mouth this is usually knife-edged and fills the interdental space.
7. Muco-gingival junction - The scalloped line that divides the attached gingiva from the alveolar mucosa.

### Periodontal biotypes

A gingival thickness of ≥2 mm is defined as thick biotype and a gingival thickness of <1.5 mm as thin biotype. Various invasive and non-invasive methods have been proposed to measure tissue thickness. These include direct measurement, probe transparency method, ultrasonic devices, and cone-beam computed tomography scan. Furthermore, placing a periodontal probe in the gingival sulcus and observing the transparency is a simple method to determine tissue thickness.

Many methods are proposed to measure gingival thickness. The gingival thickness can e.g., be assessed by the direct method, Probe transparency (TRAN) method, Ultrasonic devices and/or Cone Beam Computed Tomography (CBCT) scans.

The term periodontal biotype categorizes the gingiva into "thick-flat" and "thin-scalloped" biotypes. Thick gingival tissue is associated with a broad zone of the keratinized tissue and flat gingival contour suggestive of thick bony architecture and also is more resistant to inflammation and trauma. Thin gingival tissue is associated with a thin band of the keratinized tissue, scalloped gingival contour suggestive of thin bony architecture and is more sensitive to inflammation and trauma. Inflammation of the periodontium results in increased pocket formation and gingival recession in thick and thin tissues respectively.

In a presently preferred aspect, a pharmaceutic dental and/or cosmetic composition according to the current invention is used in promoting reinforcement and/or thickening of gingiva in patients with a thin gingiva biotype. The composition according to the current invention can to this aspect be administered before and/or after an intended surgical procedure, such as before a placement of a dental device, or irrespective of any intended surgical procedure as a general improvement of the gingival biotype. In the latter case, the use of the current composition is to improve the function of the patient's gingiva, such as to prevent a disease or disorder, or to improve the cosmetic appearance of the patient's gingiva.

A pharmaceutic dental and/or cosmetic composition according to the current invention can be used in treating a gingival deficiency and/or disorder selected from the group consisting of gingival thin biotype, gingival recession and enlargement and gingival papilla regeneration.

A pharmaceutic dental and/or cosmetic composition according to the current invention can be used in promoting gingival sealing around dental implants (periimplantitis prevention and treatment) and/or alveolar ridge preservation.

### Periodontal ligament (PDL)

The periodontal ligament, commonly abbreviated as the PDL, is a group of specialized connective tissue fibres that essentially attach a tooth to the alveolar bone within which it sits. It inserts into root cementum one side and onto alveolar bone on the other.

The PDL consists of principal fibres, loose connective tissue, blast and clast cells, oxytalan fibres and Cell Rest of Malassez.

The PDL is a part of the periodontium that provides for the attachment of the teeth to the surrounding alveolar bone by way of the cementum.

Administration of a composition according to the current invention improves the function of the periodontal ligament of the patient.

### Supracrestal attached tissue

The supracrestal attached tissues are histologically composed of the junctional epithelium and supracrestal connective tissue attachment. Infringement within the supracrestal connective tissue attachment is associated with inflammation and loss of periodontal supporting tissue.

There are two types of gingivae that are clearly recognizable, and they are known as the marginal gingiva that is mobile, and the attached gingiva.

In one aspect, the current invention relates to the use of a pharmaceutical, dental and/or cosmetic composition according to the current invention for improving soft tissue healing and/or soft tissue regeneration of the supracrestal epithelium and the supracrestal connective tissue at the placement site of the device.

Such a use improves in particular the soft tissue healing and/or soft tissue regeneration of the junctional epithelium, the sulcular epithelium and the supracrestal connective tissue at the placement site of the device at the placement site of said percutaneous dental device.

### Biological width

The natural barrier that develops around the teeth and dental implants to protect the alveolar bone from disease and infection is known as the biologic width. As such, biologic width is vital for the preservation of periodontal health and removal of irritation that could damage the periodontium.

The biologic width can be identified by probing under local anaesthesia to the bone level (referred to as "sounding to bone") and subtracting the sulcus depth from the resulting measurement. If this distance is less than 2 mm at one or more locations, a diagnosis of biologic width violation can be confirmed. Increased probing depths have been associated with increased risk of peri-implant disease and implant failure.

The biologic width is unique to each patient and will typically vary from about 0.75-4.3 mm. The mean biologic width is 2.04 mm. Of this, the connective tissue attachment is 1.07 mm and about 0.97 mm is occupied by the junctional epithelium.

Administration of a pharmaceutical, dental and/or cosmetic composition according to the current invention improves regrowth and/or regeneration of the biological width, either before or after the placement of a percutaneous dental device.

Since, as discussed above, the biologic width is unique to each patient, an improved regrowth of the biological width needs to be determined in relation to the natural biological width of the patient and can thus be from about 0.75-4.3 mm. As a rule, to a mean value of more than 2 mm.

### Wound healing in soft tissue

Soft tissue healing is defined as the replacement of destroyed tissue by living tissue in the body. This process consists of two parts - regeneration and repair. There are no defined boundaries between stages as the wound healing response "transitions" into the next stage of healing.

Oral wounds follow a similar pattern. The tissue specificities of the gingival, alveolar and palatal mucosa appear to be innately and not necessarily functionally determined. The granulation tissue originating from the periodontal ligament or from connective tissue originally covered by keratinized epithelium has the potential to induce keratinization. However, it also appears that deep palatal connective tissue may not have the same potential to induce keratinization as the palatal connective tissue originating from an immediately subepithelial area. Epithelial healing following non-surgical and surgical periodontal therapy appears to be completed after a period of 7-14 days. Structural integrity of a maturing wound between a denuded root surface and a soft tissue flap is achieved at approximately 14-days post-surgery. The formation of the biological width and maturation of the barrier function around transmucosal implants requires 6-8 weeks of healing.

Typical healing in dentistry refers to periodontal tissue healing which occurs differently in regenerative versus respective procedures and in the latter, in first intention versus secondary intention closure. Regenerative procedures aim to produce new periodontal tissue as in guided tissue regeneration (GTR), while the aim of respective procedures is to remodel the existent periodontal tissues in order to eliminate the pockets and to facilitate oral hygiene maintenance. In first intention procedures, soft tissue flaps are repositioned to perfectly cover the underlying hard tissue, while, in secondary intention procedures, surgical flaps are placed in close proximity to the remodelled hard tissue to allow best new soft tissue attachment. Administration of a composition according to the current invention can stimulate and improve GTR, both in first intention procedures and second intention procedures.

In dental implant surgery without bone augmentation procedures, soft tissue healing differs from the standard 2-stage procedures in which soft tissues completely cover the surgical bed to 1-stage procedures in which soft tissues are closely adapted around the implant neck which is left outside the surgical wound with a healing abutment or a provisional prosthesis. In this last condition, soft tissue healing is similar to that of the second stage of standard implant surgery performed for healing abutment connection in which wound margins are closely approximated to the abutment. In every case, a blood clot immediately fills the space between the implant cover screw or implant abutment/neck and the adjacent soft tissues, so that bleeding occurs on flap palpation through wound incisions or at the abutment-tissue margin interface during the first 2-3 days. In completely covered implants, first intention soft tissue healing occurs in about 1-2 weeks, while in all other cases the connective tissue aspect of the flap at the abutment-flap interface is visible for 2-3 days, at which point complete epithelialization of the abutment facing soft tissue occurs and, after the first 2 weeks peri-implant epithelium starts to migrate apically. A 3-4 mm high mature soft tissue barrier adjacent to titanium implants with about 60% of a new epithelium attachment is completely formed within 6-8 weeks and remains stable for at least 12-15 months, possibly reaching a greater final width in procedures different from conventional 2-stage procedures with implant insertion in healed sites.

In conclusion, oral soft tissue healing at teeth, implants and the edentulous ridge follows the same phases as skin wound healing.

The current invention relates to a pharmaceutical, dental and/or cosmetic composition comprising a cell culture population of human fibroblasts (hPFB) and/or human periodontal ligament cells (hPDL) and purified and/or isolated enamel matrix derivative (EMD) proteins, either as lyophilized powder, or within a suitable pharmaceutical carrier for use in inducing and/or promoting tissue healing in the CMF, such as in the oral gingiva.

In summary, the use of the herein described composition induces and/or promotes novel formation of soft tissue in the oral cavity and/or the craniomaxillofacial complex (CMF), for use in promoting reinforcement and/or thickening of gingiva in patients with a thin gingiva biotype, for use in treating a gingival deficiency and/or disorder selected from the group consisting of gingival thin biotype, gingival recession and enlargement and gingival papilla regeneration and/or for use in promoting gingival sealing around dental implants (periimplantitis prevention and treatment) and/or alveolar ridge preservation.

### Hard-tissue

The term "hard tissue" as used herein refers to a tissue that has become mineralized, or having a firm intercellular substance, for example, cartilage and bone. In dentistry, the term is used to denote any of the three calcified tissue components of the tooth: enamel, dentin and cementum.

In one aspect of the current invention, a pharmaceutic dental and/or cosmetic composition according to the current invention is used in extraction socket management, for treating intrabony pockets, promoting alveolar ridge regeneration (periodontology), for promoting alveolar ridge preservation and/or extraction socket management.

In this aspect, a pharmaceutic dental and/or cosmetic composition according to the current invention can be used in combination with an implantable 3D scaffold, such as a 3D scaffold-sponge.

### Bone

The term "bone" as used herein refers to a hard connective tissue consisting of cells embedded in a matrix of mineralized ground substance and collagen fibres. The fibres are impregnated with a form of calcium phosphate similar to hydroxyapatite as well as with substantial quantities of carbonate, citrate, and magnesium. Bone consists of a dense outer layer of compact substance or cortical substance covered by the periosteum and an inner loose, spongy substance; the central portion of a long bone is filled with marrow.

The term "odontoblasts" as used herein refers to tall columnar cells of pulp derived from ectomesenchymal cells of neural crest origin. Odontoblasts form dentin and express dentin matrix protein 1 (DMP-1) and Dentin sialophospho- protein (DSPP).

The term "osteoblasts" as used herein refers to cells that arise when osteoprogenitor cells or mesenchymal cells, which are located near all bony surfaces and within the bone marrow, differentiate under the influence of growth factors. Osteoblasts, which are responsible for bone matrix synthesis, secrete a collagen rich ground substance essential for later mineralization of hydroxyapatite and other crystals, called osteoid. Osteoblasts cause calcium salts and phosphorus to precipitate from the blood, which bond with the newly formed osteoid to mineralize the bone tissue. Once osteoblasts become trapped in the matrix they secrete, they become osteocytes. From least to terminally differentiated, the osteocyte lineage is (i) Colony-forming unit-fibroblast (CFU-F); (ii) mesenchymal stem cell/marrow stromal cell (MSC); (iii) osteoblast; and (iv) osteocyte.

The term "osteoclast" as used herein refers to large multinucleate cells associated with areas of bone resorption (breakdown).

The term "osteoconduction" as used herein refers to a process by which bone is directed so as to conform to a material's surface. An osteoconductive environment facilitates the spontaneous formation of bone. An osteoconductive surface is one that permits bone growth on its surface or down into pores, channels, or pipes. Osteoconductive mate- rial facilitates the spontaneous formation of bone by furnishing a microenvironment that supports the ingrowth of blood vessels, perivascular tissue, and osteoprogenitor cells into the site where it is deposited. Examples of osteoconductive materials, include, without limitation, the particulate granular matrix of the described invention.

The term "osteogenesis" as used herein refers to the development or formation of new bone by bone forming or osteocompetent cells.

The term "osteoinduction" as used herein refers to a process by which primitive, undifferentiated, and pluripotent cells are stimulated to develop into a bone forming cell lineage thereby inducing osteogenesis.

The terms "osteoinductive components" and "osteogenic factors" are used interchangeably to refer to the plethora of mediators associated with bone development and repair, including, but not limited to, bone morphogenic proteins (BMPs), vascular endothelial growth factor (VEGF), basic fibroblast growth factor (bFGF), transforming growth factor beta (TGF-|3), and platelet-derived growth factor (PDGF).

The term "osteointegration" refers to an anchorage mechanism whereby nonvital components can be incorporated reliably into living bone and that persist under all normal conditions of loading.

### Bone graft

The term "bone graft" as used herein refers to bone transplanted from a donor site to a recipient site, without anastomosis of nutrient vessels. Bone can be transplanted within the same individual (i.e., autogenetic graft) or between different individuals (i.e., allogeneic graft).

### Cementum

The cementum is a specialized calcified substance covering the root of a tooth. The cementum is the part of the periodontium that attaches the teeth to the alveolar bone by anchoring the periodontal ligament. Sharpey fibres are part of the principal collagenous fibres of the periodontal ligament embedded in the cementum and alveolar bone to attach the tooth to the alveolus.

If cementum can be observed on teeth, it can imply that the roots are exposed, showing that the clinical crown (the exposed part of the tooth) is bigger than the anatomical crown (the surface of the tooth covered by enamel). This is often due to gingival recession and may be an indication of periodontal disease.

Administration of a composition according to the current invention can reduce gingival recession and/or restore a healthy gingiva in patients suffering from gingival recession and/or periodontal disease.

### Scaffolds

A pharmaceutic dental and/or cosmetic composition according to the current invention can be used in combination with an implantable 3D scaffold, such as a 3D scaffold-sponge.

### Cultures of human soft tissue cells

The current invention relates to a pharmaceutical, dental and/or cosmetic composition comprising a cell culture population of human fibroblasts (hPFB) and/or human periodontal ligament cells (hPDL).

### Cell culture populations

The basic building block of all reconstruction starts with the cell. To develop tissues of skin and/or mucosa, the cells must be of ectodermal origin, specifically keratinocytes. Recent advances in cell culturing have developed protocols to growth keratinocytes, necessary for skin and mucosa, without the use of serum, irradiated feeder layers, and pituitary extract; thus, paving the way for their use in clinical applications.

In general, the cell culture population can comprise one or more of human fibroblasts or human periodontal ligament cells.

The cells can be based on the patients' own autogenous cells, which are then purified and cultivated.

In one embodiment, the pharmaceutic dental and/or cosmetic composition according to the current invention comprises a cell culture population of human fibroblasts (hPFB). Currently preferred, the cell culture population of human fibroblasts (hPFB) are oral human fibroblasts (hPFB).

In another embodiment, the pharmaceutic dental and/or cosmetic composition according to the current invention comprises a cell culture population of human periodontal ligament cells (hPDL).

In another embodiment, the pharmaceutic dental and/or cosmetic composition according to the current invention comprises a cell culture population of human fibroblasts (hPFB) and a cell culture population of human periodontal ligament cells (hPDL).

In some embodiments, the cell culture population is a population of autologous, expanded fibroblasts and/or periodontal ligament cells.

According to some embodiments, the population of fibroblasts is of dermal origin (meaning of or relating to skin).

According to some such embodiments, the population of cells is allogeneic. According to some embodiments, the population of cells secretes at least one growth-inductive factor. According to some embodiments, the population of cells is capable of regenerating a target tissue.

The type of cell culture medium and the method for culturing cells is not particularly limited. A number of base media are known in the art of mammalian cell culture, such as Dulbecco's Modified Eagle Media (DMEM), Knockout- DMEM (KO-DMEM), and DMEM/F12, although any base 50 medium that supports the growth of cells can be employed.

According to some embodiments, the cell culture medium is biologically compatible with the subject in that the cell culture medium that is used to culture cells does not contain any components that would be expected to negatively affect the health of the subject after administration of the induced cells. Thus, an appropriate cell culture medium can also comprise one or more serum-free medium supplements, i.e., a supplement that can be added to a medium to replace some or all of the serum that would normally be added to the medium to support the propagation and/or maintenance of cells in culture.

According to some embodiments, the cells can be grown using a cell expansion system (CES), which can be used to grow, expand, and/or differentiate a variety of cell types that may be used for both research and therapeutic purposes. An exemplary CES system is disclosed in US20180142199. According to some embodiments, the CES is a TerumoBCT Quantum^{®} CES.

### Fibroblast

The term "fibroblast" as used herein refers to a connective tissue cell that makes and secrets collagen protein. Fibroblasts, the most common cell type found in connective tissues, play an important role in healing wounds. Like other cells of connective tissue, fibroblasts are derived from primitive mesenchyme. They are essential players of soft tissues ECM synthesis (collagen).

### Stem cells

The term "stem cells" refers to undifferentiated cells having high proliferative potential with the ability to self- renew that can generate daughter cells that can undergo terminal differentiation into more than one distinct cell phenotype.

As used herein, the terms "osteoprogenitor cells," "mesenchymal cells," "mesenchymal stem cells (MSC)," or "marrow stromal cells" are used interchangeably to refer to multipotent stem cells that differentiate from CFU-F cells capable of differentiating along several lineage pathways into osteoblasts, chondrocytes, myocytes and adipocytes. When referring to bone or cartilage, MSCs commonly are known as osteochondrogenic, osteogenic, chondrogenic, or osteoprogenitor cells, since a single MSC has shown the ability to differentiate into chondrocytes or osteoblasts, depending on the medium.

### Multipotent

The term "multipotent" as used herein refers to a cell capable of giving rise to a limited number of cell types of a particular cell line.

### Pluripotent

The term "pluripotent' as used herein refers to the ability to develop into multiple cells types, including all three embryonic lineages, forming the body organs, nervous sys- tem, skin, muscle, and skeleton. A "pluripotent stem cell" or "pluripotent cell" is a cell that has the ability under appro- priate conditions of producing progeny of several different cell types that are derivatives of all of the three germinal layers (endoderm, mesoderm, and ectoderm). Examples of pluripotent stem cells are embryonic stem (ES) cells, embryonic germ stem (EG) cells, embryonic Carcinoma (EC) cells, induced pluripotent stem (iPS) cells, and adult stem cells. PSCs cells may be derived from any organism of interest, including, primate, e.g., human, canine, feline, murine, equine, porcine, avian, camel, bovine, ovine, etc.

The term "progenitor cell" as used herein refers to an early descendant of a stem cell that can only differentiate, but can no longer renew itself. Progenitor cells mature into precursor cells that mature into mature phenotypes. Flema- topoietic progenitor cells are referred to as colony-forming units (CFU) or colony-forming cells (CFC). The specific lineage of a progenitor cell is indicated by a suffix, such as, but not limited to, CFU-E (erythrocytic), CFU-F (fibroblastic), CFU-GM (granulocytic/macrophage), and CFU-GEMM (pluripotent hematopoietic progenitor). Osteoclasts arise from hematopoietic cells of the monocyte/neutrophil lineage (CFU-GM). Osteoprogenitor cells arise from mesenchymal stem cells and are committed to an osteocyte lineage.

The term "propagate" as used herein refers to reproduce, multiply, or to increase in number, amount, or extent by any process.

Methods for producing such cells are known in the art.

### allogeneic

The term "allogeneic" as used herein refers to being genetically different although belonging to or obtained from the same species. The term "allogeneic graft" or "allograft" as used herein refers to a tissue that is grafted into or on the body of a recipient, where the recipient is an individual different from the donor that provides the source tissue but with both the recipient and the donor being of the same species.

### Autologous

The terms "autologous" or "autogeneic" as used herein mean derived from the same organism. The terms "autologous graft", "autogeneic graft", "auto- plastic graft" or "autograft" as used herein refers to a tissue that is grafted into a new position in or on the body of the same individual.

### Enamel matrix derivative (EMD) proteins

The current invention relates to a pharmaceutical, dental and/or cosmetic composition comprising purified and/or isolated enamel matrix derivative (EMD) proteins, which have a molecular weight between 1-160kDa.

In one embodiment of the herein described pharmaceutic dental and/or cosmetic composition, the EMD proteins are isolated porcine Enamel Matrix Derivative (EMD) proteins. Typically, the EMD proteins comprise amelogenins, enamelin, tuft protein, proteases and albumin. In one embodiment, at least 80% of the EMD proteins are amelogenins.

### EMD

Enamel matrix proteins, present in the enamel matrix, are most well-known as precursors to enamel. Prior to cementum formation, enamel matrix proteins are deposited on the root surface at the apical end of the developing tooth-root. There is evidence that the deposited enamel matrix is the initiating factor for the formation of cementum. Again, the formation of cementum in itself is associated with the development of the periodontal ligament and the alveolar bone. Enamel matrix proteins can therefore promote periodontal regeneration through mimicking the natural attachment development in the tooth (Gestrelius S, Lyngstadaas SP, Hammarstrom L. Emdogain - periodontal regeneration based on biomimicry. Clin Oral Invest 4:120-125 (2000)).

Isolated enamel matrix proteins (in the current context called enamel matrix derivative (EMD) proteins) are able to induce not only one, but an orchestrated cascade of factors, naturally found in tissues developing adjacent to the enamel matrix. They mimic the natural environment of a developing tissue and thus mimic a natural stimulation for tissue regeneration, cell differentiation and/or maturation.

Enamel matrix derivative (EMD), in the form of a purified acid extract of proteins from pig enamel matrix, has previously been successfully employed to restore functional periodontal ligament, cementum and alveolar bone in patients with severe tooth attachment loss (Hammarstrbm et al., 1997, Journal of Clinical Periodontology 24, 658-668).

Enamel Matrix Derivative (EMD) proteins are widely used in clinical dentistry because of their ability to promote regeneration of soft and hard tissues and to reduce inflammation and infections.

Purified Enamel Matrix Derivative (EMD) proteins contain 3 major protein fractions which are separable by High Pressure Liquid Chromatography (HPLC). These fractions are named fraction A, B and C, respectively. During separation by gel filtration, proteins in EMD separate into 3 distinct peaks as defined by the protein concentration in the eluate. The first peak contains high molecular weight proteins (100 kDa -160kDa) and is associated with the void volume. The second protein peak elutes at approximately 50 kDa, while proteins in the third peak are extrapolated from the standard curve to have molecular weights of approximately 10 kDa. Atypical weight ratio of the isolated and/or purified proteins is about 80/8/12 between the main protein peaks at 20, 14 and 5 kDa, respectively.

EMD proteins, as enamel matrix proteins, are composed of a number of proteins, such as amelogenin, enamelin, tuft protein, proteases, and albumin. Amelogenin, a major constituent of EMD proteins and/or enamel matrix proteins (up to approximately 90%, such as at least 80%), are a family of hydrophobic proteins derivable from a single gene by alternative splicing and controlled post secretory processing. They are highly conserved throughout vertebrate evolution and demonstrate a high overall level of sequence homology among all higher vertebrates examined (80%). In fact, the sequences of porcine and human amelogenin gene transcript differ only in 4% of the bases. Thus, enamel matrix proteins or EMD proteins, although of porcine origin, are considered "self" when encountered in the human body and can promote dental and soft tissue restoration and/or regeneration in humans without triggering allergic responses or other undesirable reactions.

In the present context, purified Enamel Matrix Derivative (EMD) proteins are thus defined as isolated and/or purified enamel matrix proteins comprising at least 60-80% amelogenin, such as at least 60, 61, 62, 63, 64, 65, 66, 67, 68, 69, 70, 71, 72, 73, 74, 75, 76, 77, 78, 79 or 80 % amelogenin, with a molecular weight of about 20-25 kDa, such as 20, 21,22, 23, 24, or 25 kDa, or such as between 20-22, 20-24, or 20-23 kDa.

In general, the weight ratio of the purified and/or isolated enamel matrix proteins is about 80/8/12, such as 75-85/5-12/5-15, or such as at least 80%, at least 8%, and at least 5%, between the main protein peaks of fraction A, B and C, respectively. Approximately 60-90%, such as at least 60, 61, 62, 63, 64, 65, 66, 67, 68, 69, 70, 80, 70-90, 60-70, 70-80, or 80-90% of the purified and/or isolated enamel matrix proteins are amelogenin and/or fragments or derivatives of amelogenin.

Methods to determine identity and similarity of peptide, proteins, amino acid sequences or nucleic acid sequences are codified in publicly available programs. Preferred computer program methods to determine identity and similarity between two sequences include, but are not limited to, the GCG program package (Devereux, J et al (1994)) BLASTP, BLASTN, and FASTA (Altschul, S.F. et al (1990)). The BLASTX program is publicly available from NCBI and other sources (BLAST Manual, Altschul, S.F. et al, Altschul, S.F. et al (1990)). Another preferred example is Clustal W (http://www.ebi.ac.uk/clustalw/). Each sequence analysis program has a default scoring matrix and default gap penalties. In general, a molecular biologist would be expected to use the default settings established by the software program used.

The amino acids in an EMD protein and/or enamel matrix protein may further be modified in terms of chemistry, isometry or in any other way as long as the sequences of the protein is intact. Modifications of the amino acids of the EMD protein and/or enamel matrix protein may increase the activity, stability, biocompatibility or clinical performance of the proteins, or reduce toxicity and adverse reactions to the proteins. Examples of chemical modifications include, but are not limited to, glycosylation and methylation. The amino acids may also be of all different types of stereoisomeric forms, such as D or L forms of amino acids, or S or R isomers. The complete amino acid sequence, individual or a plurality of amino acids in an EMD protein and/or enamel matrix protein of the invention may also be replaced by synthetic or recombinant analogues thereof. The use of synthetic analogues may e.g., result in an EMD protein and/or enamel matrix protein that is more stable and less prone to degradation. Examples of unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, R-alanine*, L-a-amino butyric acid*, L-g-amino butyric acid*, L-a-amino isobutyric acid*, L-e-amino caproic acid#, 7-amino heptanoic acid*, L-methionine sulfone#*, L-norleucine*, L-norvaline*, p-nitro-L-phenylalanine*, L-hydroxyproline#, L-thioproline*, methyl derivatives of phenylalanine (Phe) such as 4-methyl-Phe*, pentamethyl-Phe*, L-Phe (4-amino)#, L-Tyr (methyl)*, L-Phe (4-isopropyl)*, L-Tic (1,2,3,4-tetrahydroisoquinoline-3-carboxyl acid)*, L-diaminopropionic acid # and L-Phe (4-benzyl)*. The notation * is herein utilised to indicate the hydrophobic nature of the derivative whereas # is utilised to indicate the hydrophilic nature of the derivative, #* indicates amphipathic characteristics.

EMD proteins and/or enamel matrix proteins may further comprise N- and/or C-terminal tags comprising the amino acids His and/or Met.

In one embodiment, the EMD proteins and/or enamel matrix proteins are produced instead of isolated from a natural source, e.g., by synthetic production or biosynthesis. The EMD proteins and/or enamel matrix proteins, or fragments thereof, may be produced by any known method for production of peptides, such as synthetic production by chemical synthesis. Synthetic production also allows the use of amino acid analogues which may improve the stability of the proteins or fragments produced. The skilled person knows the methods that are available for the synthesis of an amino acid sequence.

Preferably, bioproduction, such as biosynthesis, may be used as a method for producing the EMD proteins and/or enamel matrix proteins, or fragments thereof. Bioproduction means the production of an amino acid sequence in a biological system, such as a cell culture or in microbial cells, e.g., bacterial or yeast cells.

The present invention relates to a composition comprising enamel matrix proteins and/or enamel matrix derivative (EMD) proteins, wherein at least 60-70%, such as 60, 61, 62, 63, 64, 65, 66, 67, 68, 69 or 70%, of the proteins have a molecular weight between 16-40 kDa, such as above 20 kDa, such as 16, 17, 18 or 19 kDa.

The composition comprising isolated enamel matrix derivative proteins described in the present invention comprises isolated enamel matrix proteins at a concentration of 0.01 to 100 mg/ml, such as of 0.3 mg/ml-100 mg/ml, or of 29-31 mg/ml, such as at a concentration of 29, 30 or 31 mg/ml. In one embodiment, the concentration of the EMD proteins and/or enamel matrix proteins in a composition according to the invention is around 29 mg/ml.

### Derivative

The term "derivative" as used herein means a compound that may be produced from another compound of similar structure in one or more steps. A "derivative" or "derivatives" of a peptide or a compound retains at least a degree of the desired function of the peptide or compound. Accordingly, an alternate term for "derivative" may be "functional derivative". Derivatives can include chemical modifications of the peptide, such as alkylation, acylation, carbamylation, iodination, or any modification that derivatizes the peptide. Such derivatized molecules include, for example, those molecules in which free amino groups have been derivatized to form amine hydrochlorides, p-toluene sulfonyl groups, carbobenzoxy groups, t-butyloxycarbonyl groups, chloro- acetyl groups, or formal groups. Free carboxyl groups can be derivatized to form salts, esters, amides, or hydrazides. Free hydroxyl groups can be derivatized to form O-acyl or O-alkyl derivatives. The imidazole nitrogen of histidine can be derivatized to form N-im-benzylhistidine. Also included as derivatives or analogues are those peptides that contain one or more naturally occurring amino acid derivative of the twenty standard amino acids, for example, 4-hydroxypro- line, 5-hydroxylysine, 3-methylhistidine, homoserine, orni- thine, or carboxyglutamiate, and can include amino acids that are not linked by peptide bonds. Such peptide derivatives can be incorporated during synthesis of a peptide, or a peptide can be modified by well-known chemical modification methods (see, e.g., Glazer et al., Chemical Modification of Proteins, Selected Methods and Analytical Procedures, 5 Elsevier Biomedical Press, New York (1975)).

### Emdogain^{®}

Recent studies conducted by the present inventors have developed a gel that is injectable by means of a syringe to the site of a bone defect, named Emdogain^{®}. This gel consists of two components, propylene glycol alginate (PGA) and Enamel Matrix Derivative (EMD). While PGA has a structural role and acts as a carrier, EMD is the active component that favours the regeneration of the diseased periodontal tissue by mediating the formation of acellular cementum at the root of the tooth and providing a foundation for the growth of the tissue associated with functional attachment. Once the gel is applied to the site of a defect, the pH tends to strive to the physiological value and when it reaches a value of 6, it causes EMD to precipitate. Afterwards, the osteoblast and cementoblast cells are enticed by the natural cocktail of isolated enamel matrix proteins to proliferate and cause the ligament extension from the gingival wall into the intrabony gum.

Straumann^{®} Emdogain^{®} is a commercially available product composed of Propylene Glycol Alginate (PGA) and porcine Enamel Matrix Derivative (EMD) proteins. The PGA employed in the manufacture of Straumann^{®} Emdogain^{®} has a viscosity of 50-175 mPa s (EMD in a 2 % PGA aqueous solution 22°C (Brookfield viscosity)). The composition Straumann^{®} EMDOGAIN^{®} itself, displays a viscosity of 3.0 Pa s (3000 mPa s at 22°C). Upon dissolving alginates in water, the molecules hydrate and the solution gains viscosity. The viscosity of an alginate solution depends on the concentration of alginate and the length of the alginate molecules, i.e., the number of monomer units in the chains. In general, the longer the chains, the higher the viscosity at similar concentrations. The dissolved molecules are not completely flexible; rotation around the glycosidic linkages in the G-block regions is somewhat hindered, resulting in a stiffening of the chain. Solutions of stiff macromolecules are highly viscous.

In one embodiment of the current invention, the composition comprises EMDOGAIN.

### Growth factor(s)

In one aspect, a pharmaceutic dental and/or cosmetic composition according to the current invention further comprises one or more growth factor(s).

The term "growth factor" as used herein refers to extra- cellular polypeptide molecules that bind to a cell-surface receptor triggering an intracellular signalling pathway, leading to proliferation, differentiation, or other cellular response. Growth factors are specialized polypeptide molecules that bind to receptors on target cells and deliver messages regarding migration, proliferation, differentiation, survival and secretion. Growth factors include, but are not limited to, cytokines and hormones.

Growth factors contemplated to be included in the composition of the current invention are for example, Bone morphogenetic proteins (BMPs), Fibroblast Growth Factors (FGF), angiogenic factors, such as Insulin-Like Growth Factor (IGF), platelet-derived growth factor PDGF, Hepatocyte growth factor (HGF), TGFm Angiopoeitin-1, and stem cell factor (SCF), Insulin-Like Growth Factor (IGF-1), Transforming Growth Factor Beta (TGF-|3), Neural Epidermal Growth-Factor-Like 1 (NELLI), Vascular endothelial growth factor (VEGF), Platelet-derived growth factor (PDGF), IL-lalpha, IL-1 beta, Epidermal growth factor (EGF), Fibronectin, keratinocyte growth factor (KGF), transforming growth factor (TGF), and tumor necrosis factor (TNF).

In one embodiment, a composition according to the current invention comprises one or more growth factors selected from the list consisting of EGF, FGF, KGF, PDGF, TGF, TNF and VEGF.

In one embodiment, a composition according to the current invention comprises one or more growth factors selected from the list consisting of BMP-2 and 4, VEGF, bFGF, TGFbeta, and PDGF.

### bFGF

The term "basic fibroblast growth factor" or "bFGF" as used herein refers to a multifunctional effector for many cells of mesenchymal and neuroectodermal origin that is a potent inducer of neovascularization and angiogenesis.

In one embodiment, a composition according to the current invention comprises bFGF.

### Suitable pharmaceutical carriers

One embodiment of the current invention relates to a pharmaceutical, dental and/or cosmetic composition comprising:
a. a cell culture population of human fibroblasts (hPFB) and/or human periodontal ligament cells (hPDL),
b. purified and/or isolated enamel matrix derivative (EMD) proteins, which have a molecular weight between 1-160kDa, and
c. a suitable pharmaceutical carrier.

Typically, the carrier has a pH between 6-7.5 such as between 6.0 and 7.0, suchas between 7.0-7.5, such as between 7.0 and 7.25, such as a pH of 6.0, 6.5, 6.75, 7.0, 7.1, 7.2, 7.3, 7.4, 7.5, 7.6, 7.7, or 7.5, such as no more than 7.5, such as no less than 5.0.

In one embodiment, a pharmaceutic dental and/or cosmetic composition according to the current invention is formulated as a solution, such as a liquid and/or an aqueous fluid.

In one embodiment, a pharmaceutic dental and/or cosmetic composition according to the current invention, the carrier is EDTA or an alginate carrier, such as but not limited to Propylene Glycol Alginate (PGA), a hydrogel, Hyaluronic Acid and/or a poloxamer.

In a currently preferred embodiment, a pharmaceutic dental and/or cosmetic composition according to the current invention is formulated as an injectable gel.

In one embodiment, the suitable pharmaceutical carrier of the composition comprising isolated enamel matrix proteins is to be selected from the group consisting of acetic acid and PBS. In one embodiment, the suitable pharmaceutical carrier of the composition comprising isolated enamel matrix proteins is acetic acid.

In one embodiment, the suitable pharmaceutical carrier of the composition comprising EMD proteins is to be selected from the group consisting of acetic acid and PBS. In a presently preferred embodiment, the suitable pharmaceutical carrier of the composition comprising isolated enamel matrix proteins is acetic acid.

### Carrier

The term "carrier" as used herein refers to a pharmaceutically acceptable inert agent or vehicle for delivering one or more active agents to a subject, and often is referred to as "excipient". The carrier must be of sufficiently high purity and of sufficiently low toxicity to render it suitable for administration to the subject being treated. The carrier further should maintain the stability and bioavailability of an active agent.

The composition may be formulated according to conventional pharmaceutical practice, see, e.g., "Remington's Pharmaceutical Sciences" and "Encyclopedia of Pharmaceutical Technology", edited by Swarbrick, J. & J. C. Boylan, Marcel Dekker, Inc., New York, 1988. In the present context, pharmaceutically or cosmetically acceptable excipient, carrier and/or diluent is a substance which is substantially harmless to the individual to which the formulation is to be administered. Such an excipient, carrier and/or diluent normally fulfills the requirements given by the national health authorities. Official pharmacopoeias such as e.g. the British Pharmacopoeia, the United States of America Pharmacopoeia and The European Pharmacopoeia set standards for pharmaceutically acceptable excipients.

The suitable pharmaceutical carrier of the present invention may include solvents, buffering agents, preservatives, humectants, chelating agents, antioxidants, stabilizers, emulsifying agents, suspending agents, gel-forming agents, ointment bases, penetration enhancers, perfumes, and skin protective agents.

Examples of buffering agents are e.g. citric acid, acetic acid, tartaric acid, lactic acid, hydrogenphosphoric acid, etc.

### Sodium carboxyl methylcellulose (CMC)

Carboxymethyl cellulose or cellulose gum is a cellulose derivative with carboxymethyl groups bound to some of the hydroxyl groups of the glucopyranose monomers that make up the cellulose backbone. It is often used in its sodium salt form, sodium carboxymethyl cellulose. Sodium carboxymethyl cellulose (CMC) is one of the most important products of cellulose ethers, which are formed by natural cellulose modification as a kind of cellulose derivate with an ether structure. Due to the fact that the acid form of CMC has poor water solubility, it is usually preserved as sodium carboxymethylcellulose, which is widely used in many industries and regarded as monosodium glutamate in industry. CMC could be used as flocculating agent, chelating agent, emulsifier, thickening agent, water-retaining agent, sizing agent, and film-forming material, and so on. CMC is also widely applied in fields such as electronics, pesticides, leather, plastics, printing, ceramics, and the daily-use chemical industry. Moreover, due to its excellent properties, wide application, and the developing potential fields, CMC has broad application prospects.

In one embodiment, the suitable pharmaceutical carrier of the composition comprising EMD proteins comprises sodium carboxyl methylcellulose.

### PGA

Propylene glycol alginate (PGA) is an emulsifier, stabilizer, and thickener used in food products. It is a food additive with E number E405. Chemically, propylene glycol alginate is an ester of alginic acid, which is derived from kelp. Some of the carboxyl groups are esterified with propylene glycol, some are neutralized with an appropriate alkali, and some remain free.

A "solution" generally is considered as a homogeneous mixture of two or more substances. It is frequently, though not necessarily, a liquid. In a solution, the molecules of the solute (or dissolved substance) are uniformly distributed among those of the solvent.

The term "solvent" as used herein refers to a substance capable of dissolving another substance (termed a "solute") to form a uniformly dispersed mixture (solution).

In one embodiment, the composition of the present invention comprising isolated enamel matrix proteins may further include arginine. In particular, in a concentration range of 700 mM or lower, such as 10-700 mM, 100-700 mM or 200-700 mM is preferred, in particular when the concentration of EMD is at least 20 mg/ml, such as about 29-31 mg/ml, such as about 31 mg/ml.

### Method of preparing a composition

### Isolation of EMD proteins from developing teeth

The in the experimental section described extraction and isolation of EMD from pigs' developing teeth is one example of how EMD can be extracted. There are several other methods to prepare EMD, such as described in Hammarström et al, 1997, J. Clin Periodontol; 24: 669-677, US2013/0210735, WO201506970, or in Yasuo Yamakoshi, et.al., Methods Mol Biol. 2019; 1922: 239-250. There are several other well-known processes that are being used in the field since over 30 years.

Isolated EMD at a protein concentration is approx. 33 mg/ml is in the current context dissolved in 2% PGA and NaOH at a pH of between 6.0-7.0.

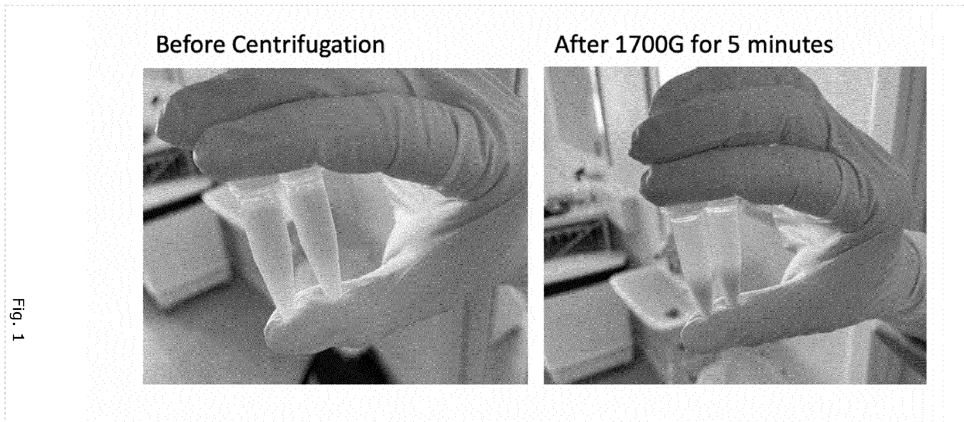

Isolated EMD in PGA/NaOH has the characteristics of a whiteish opaque (cloudy) substance with a viscosity somewhat akin to liquid honey. When loaded into a standard multiwell plate it is hard to appreciate any details of EMD apart from a thick semi-fluid mass that occludes visibility from the bottom of the well. EMD solution is then centrifuged by spinning samples of EMD at 1700G for 5 minutes wherein a part of the EMD sediment s into a pellet, leaving a somewhat less cloudy supernatant fraction above (see figure 1). The EMD supernatant consists mainly of smaller granule-like particles roughly in the size of singularized cells.

EMD in PGA/NaOH might be slightly acidic before centrifugation (pH appr. 5.0), which may have a direct impact on cells. The compositions of EMD according to the current invention have a pH slightly above 6, such as between 6.0-7.5.

### Lyophilization

One embodiment of the current invention relates to a pharmaceutic dental and/or cosmetic composition according to the current invention, wherein the cells have been lyophilized before being added to the composition and/or wherein the EMD proteins have been lyophilized before being added to the composition.

In one embodiment, a pharmaceutic dental and/or cosmetic composition according to thew current invention is provided in the form of a lyophilized powder.

### Use in medicine

A pharmaceutic dental and/or cosmetic composition according to the current invention is intended for use in medicine. One aspect of the current invention thus relates to a pharmaceutic dental and/or cosmetic composition according to the current invention is intended for use in medicine

In general, a pharmaceutic dental and/or cosmetic composition according to the current invention is used in inducing and/or promoting novel formation of soft tissue in the oral cavity and/or the craniomaxillofacial complex (CMF). As such, a pharmaceutic dental and/or cosmetic composition according to the current invention is used in promoting growth and/or healing of soft tissue in the oral cavity and/or the craniomaxillofacial complex (CMF).

In a currently preferred aspect, a pharmaceutic dental and/or cosmetic composition according to the current invention is used for promoting reinforcement and/or thickening of gingiva in patients with a thin gingiva biotype.

In another aspect, a pharmaceutic dental and/or cosmetic composition according to the current invention is used in treating a gingival deficiency and/or disorder selected from the group consisting of gingival thin biotype, gingival recession and enlargement and gingival papilla regeneration.

A pharmaceutic dental and/or cosmetic composition according to the current invention can also be for use in promoting gingival sealing around dental implants (periimplantitis prevention and treatment) and/or alveolar ridge preservation, as well as for use in extraction socket management, such as for treating intrabony pockets and/or promoting alveolar ridge regeneration (periodontology).

### Repair

The term "repair," when used in the context of the healing of damaged tissue, is defined as the restoration of tissue architecture and function after an injury. It encompasses two separate processes: regeneration and replacement. Regeneration refers to a type of healing in which new growth completely restores portions of damaged tissue to their normal state. Replacement refers to a type of healing in which severely damaged or non-regenerable tissues are repaired by the laying down of connective tissue, a process commonly referred to as scarring. While a few types of tissue injury (such as minor paper cuts) can sometimes be healed in such a way that no permanent damage remains, most of our tissue repair consists of both regeneration and replacement. Tissue repair may restore some of the original structures of the damaged tissue (such as epithelial layers) but may also result in structural abnormalities that impair organ function (such as the scar formed in the healing of a myocardial infarction).

Whether the healing of a wound proceeds down the regeneration or the replacement pathway (or both) depends, in part, on the type of tissue in which it occurs. Certain tissues of the body are more capable of cellular proliferation (and hence regeneration) than others. In this regard, there are three types of tissues: continuously dividing tissues, quiescent tissues and nondividing tissues. Continuously dividing tissues (also known as labile tissues) are comprised of cells that are constantly proliferating in order to replace dead or sloughed-off cells. Examples of such tissues include epithelia (such as skin, gastrointestinal epithelium and salivary gland tissue) and hematopoietic tissues. These tissues contain pools of stem cells, which have enormous proliferative and self-renewing ability, and which give rise to more than one type of cell. Replicating asymmetrically, each stem cell gives rise to one daughter cell that differentiates and matures and another daughter cell that remains undifferentiated and capable of beginning another self-renewing cycle.

Some tissues, known as quiescent tissues (or stable tissues) are composed of cells that normally exist in a non-dividing state but may enter the cell cycle in response to certain stimuli, such as cell injury. Tissues falling into this category include parenchymal cells of the liver, kidney and pancreas, mesenchymal cells such as fibroblasts and smooth muscle cells, endothelial cells and lymphocytes. A few types of tissue are composed of cells that have left the cell cycle permanently and are therefore unable to proliferate. These nondividing tissues (or permanent tissues) include cardiac and skeletal muscle. Tissue repair in these tissues always leaves permanent evidence of injury, such as a scar.

### Regeneration

The term "regeneration" or "regenerate" as used herein refers to a process of recreation, reconstitution, renewal, revival, restoration, differentiation, and growth to form a tissue with characteristics that conform with a natural counterpart of the tissue. Especially, embryonic and mesenchymal stem cells are attractive resources for tissue regeneration, due to their potential for the differentiation of various tissue cells in response to signal transduction mediated by cytokines. Although the exact factor responsible for a stem cell's ability to differentiate between specific cells to generate specific tissue is not fully understood yet, delivering stem cells into the body provides a strong potential for the regeneration of tissue.

### Restoration

The term "restoration" is herein used to describe the self-regeneration of bodily defects by host tissue.

### Tissue engineering

Tissue engineering is defined as "the reconstitution of tissues and organs, in vitro, for use as model systems in basic and applied research or for use as grafts to replace damaged, or diseased body parts or body functions." In the field of Oral and Maxillofacial Surgery, this is applied through the reconstructive efforts needed for the treatment of trauma, tumour resection, and to repair congenital deformities.

The term "treat" or "treating" includes abrogating, substantially inhibiting, slowing, or reversing the progression of a disease, condition, or disorder; substantially ameliorating clinical or esthetical symptoms of a condition; substantially preventing the appearance of clinical or esthetical symptoms of a disease, condition, or disorder; and protecting from harmful or annoying symptoms. Treating further refers to accomplishing one or more of the following: (a) reducing the severity of the disorder; (b) limiting development of symptoms characteristic of the disorder(s) being treated; (c) limiting worsening of symptoms characteristic of the disorder being treated; (d) limiting recurrence of the disorder(s) in patients that have previously had the disorder); and (e) limiting recurrence of symptoms in patients that were previously asymptomatic for the disorder(s).

### Minimally-invasive surgery

The current invention relates to a pharmaceutic dental and/or cosmetic composition according to the current invention for use in minimally-invasive surgery.

In the current context, the term "minimally-invasive surgery" is used to describe any surgery that is done using small incisions (cuts) and few or no stitches. During minimally invasive surgery, one or more small incisions may be made in the body. A laparoscope (thin, tube-like instrument with a light and a lens for viewing) can be inserted through one opening to guide the surgery. Tiny surgical instruments are inserted through other openings to do the surgery. Minimally invasive surgery may cause less pain, scarring, and damage to healthy tissue, and the patient may have a faster recovery than with traditional surgery.

In the current context, the terms "minimally invasive procedures" and "minimally invasive surgeries" are used interchangeably. They encompass surgical techniques that limit the size of incisions needed, thereby reducing wound healing time, associated pain, and risk of infection. Surgery by definition is invasive, and many operations requiring incisions of some size are referred to as open surgery. Incisions made during open surgery can sometimes leave large wounds that may be painful and take a long time to heal. Advancements in medical technologies have enabled the development and regular use of minimally invasive procedures.

Many conditions once requiring surgery can now be treated non-surgically.

Diagnostic techniques that do not involve incisions, puncturing the skin, or the introduction of foreign objects or materials into the body are known as non-invasive procedures. Several treatment procedures are classified as non-invasive. A major example of a non-invasive alternative treatment to surgery is radiation therapy.

Laparoscopy is an operation performed in the abdomen or pelvis using small incisions with the aid of a camera. The laparoscope aids diagnosis or therapeutic interventions with a few small cuts in the abdomen.

In one aspect, a composition according to the current invention is administered during a minimally invasive surgery, either as a powder, a liquid, a fluid, or a gel.

### Administration by injection

A pharmaceutic dental and/or cosmetic composition according to the current invention can be administered by injection, such as, but not limited to, by hydrodissection.

In one aspect, a pharmaceutic dental and/or cosmetic composition according to the current invention is administered by injection into the soft tissue in the oral cavity and/or the craniomaxillofacial complex (CMF) of a patient in need thereof.

In the current context, the injection of a composition according to the current invention into the soft tissue of a patient in need thereof is considered a minimally-invasive surgery. In that aspect, the composition is formulated as a liquid, fluid or as an injectable gel and is administered with a syringe.

In a presently preferred aspect, a pharmaceutic dental and/or cosmetic composition according to the current invention is administered by injection into the gingiva of a patient in need thereof.

### Methods of treatment

In one aspect, the current invention relates to a method of treating a patient suffering from a gingival deficiency and/or disorder selected from the group consisting of gingival thin biotype, gingival recession and enlargement and gingival papilla regeneration, wherein said patient is injected with a composition according to the current invention.

### Use for the manufacture of a pharmaceutical composition

A composition according to the current invention is in one aspect used for the manufacture of a pharmaceutical composition for use in treating a patient suffering from a gingival deficiency and/or disorder selected from the group consisting of gingival thin biotype, gingival recession and enlargement and gingival papilla regeneration.

### A kit

In one embodiment of the present invention, a kit is provided comprising:
a. a cell culture population of human fibroblasts (hPFB) and/or human periodontal ligament cells (hPDL), and
b. lyophilized purified and/or isolated enamel matrix derivative (EMD) proteins, which have a molecular weight between 1-160kDa, and
c. optionally EMC proteins selected from the group consisting of collagen, fibronectin and proteoglycans.

Said kit can further comprise:
d. a suitable pharmaceutical carrier.

Said kit can further comprise:
e. a syringe for administering the composition.

Said kit can further comprise:
f. a leaflet describing the intended use and mode of administration.

In a currently preferred embodiment, a kit according to the current invention comprises oral human fibroblasts (hPFB) and isolated porcine Enamel Matrix Derivative (EMD) proteins.

In one aspect, a kit according to the current invention is used for producing and/or manufacturing a pharmaceutic dental and/or cosmetic composition in in the form of a powder, an injectable gel, an injectable solution, a liquid and/or an aqueous fluid, for use according to the current invention.

A suitable pharmaceutical carrier in a kit according to the current invention preferably has a neutral pH, such as a pH between 7-7.75.

### Implantable 3D scaffold

In one aspect, a pharmaceutic dental and/or cosmetic composition according to the current invention is for use in combination with an implantable 3D scaffold, such as a 3D scaffold-sponge.

In that aspect, a kit according to the current invention comprises an implantable 3D scaffold, such as a 3D scaffold-sponge.

### Other embodiments

It is to be understood that while the present invention has been described in conjunction with the detailed description thereof, the foregoing description is intended to illustrate and not limit the scope of the invention, which is defined by the scope of the appended claims.

Other aspects, advantages, and modifications are within the scope of the following claims.

### Experimental section

The present invention is further illustrated by the following non-limiting experiments.

### Experiment 1

### Introduction

The main objective of the project is to provide an initial assessment of the biological compatibility and effects of Enamel Matrix Derivate (EMD) on Human Oral Fibroblasts (HOrF), and to provide an early assessment regarding the feasibility and potential benefit of in vivo EMD administration by use of a syringe.

### Project Scope

- Expansion and stock production of HOrF for experimentation
- Assessment and testing of EMD effects on HOrF
- Feasibility prediction regarding co-injection of EMD and HOrF into tissue

### Expansion and stock production of HOrF for experimentation

To produce a larger amount of HOrF needed for carrying out the tests required for the project a cryovial of commercially available HOrF cells (Innoprot. Human Oral Fibroblasts. Batch: #22095 (P10868)) was thawed and expanded (see project day reports for procedural details).

In total, 292.000 initial HOrF was expanded to a final amount of 32.200.000 HOrF distributed into 30 cryogenically stored stock vials. During the expansion the cultivated cells were monitored for confluency and phenotypic characteristics using brightfield microcopy.

### Preparation of EMD from porcine mandibular unerupted tooth buds:

EMD solution is prepared as described by Hammarström et al, 1997, J. Clin Periodontol; 24: 669-677, but for the following: the enamel matrix is not scraped from the tooth buds and the purified solution is not lyophilized, instead the solution is concentrated to about 33 mg/ml EMD protein. EMD solution can also be prepared as described in US2013/0210735, or in WO201506970.

Enamel matrix derivative (EMD) is prepared by extraction from porcine mandibular unerupted tooth buds according to the following steps:
1. Extraction under stirring in 0.5 M acetic acid at a temperature below 10C for 7 - 17 h the pH of the solution is between in the range of 2.3-2.4
2. Centrifugation to remove debris generated during extraction
3. Volume reduction to about 1/3 third of the initial volume by ultrafiltration
4. Purification by diafiltration with diluted acetic acid and water, the pH of the solution is between 4.9 to 5
5. Heat treatment at 80C for 3h to inactivate enzymes
6. Volume reduction by means of ultrafiltration until protein concentration is approx. 33 mg/ml
7. Sterile filtration

Isolated EMD at a protein concentration of approx. 33 mg/ml is in the current context dissolved in 2% sterilized PGA and NaOH at a pH of between 6.0-7.0.

Isolated EMD in PGA/NaOH has the characteristics of a whiteish opaque (cloudy) substance with a viscosity somewhat akin to liquid honey. When loaded into a standard multiwell plate it is hard to appreciate any details of EMD apart from a thick semi-fluid mass that occludes visibility from the bottom of the well. EMD solution is then centrifuged by spinning samples of EMD at 1700G for 5 minutes wherein a part of the EMD sediment s into a pellet, leaving a somewhat less cloudy supernatant fraction above (See figure 1). The EMD supernatant consists mainly of smaller granule-like particles roughly in the size of singularized cells.

EMD in PGA/NaOH might be slightly acidic before centrifugation (pH appr. 5.0), which may have a direct impact on cells. The compositions of EMD according to the current invention have a pH slightly above 6, such as between 6.0-7.5.

### Assessment and testing of EMD effects on HOrF

Before proper cell trials began, the characteristics of EMD were assessed to better predict and plan proper experimental parameters.

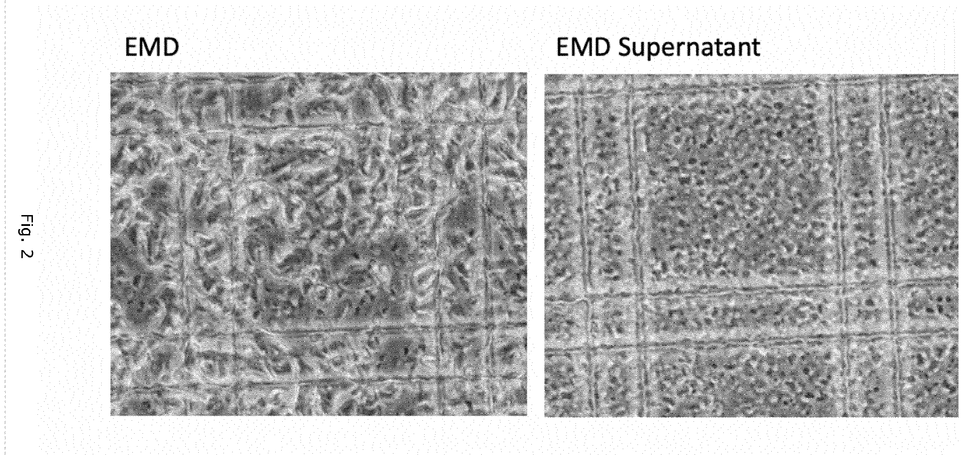

Upon inspection, EMD have the characteristics of a whiteish opaque (cloudy) substance with a viscosity somewhat akin to liquid honey. When loaded into a standard multiwell plate it is hard to appreciate any details of EMD apart from a thick semi-fluid mass that occludes visibility from the bottom of the well. Since the EMD displayed these characteristics, it was decided to attempt to centrifuge the EMD to evaluate if a less cloudy fraction could be collected. By spinning samples of EMD at 1700G for 5 minutes it was indeed observed that part of the EMD may sediment into a pellet, leaving a somewhat less cloudy supernatant fraction above (See figure 1). By loading unfractionated EMD and EMD supernatant into a standardized counting chamber it was possible to better visualize the microscopic characteristics of EMD (see figure 2).

Unfractionated EMD was demonstrated to consist of a heterogenous mixture of gel-like structures of various shapes and sizes, while EMD supernatant was instead consisted mainly of smaller granule-like particles roughly in the size of singularized cells.

Another characteristic of EMD worth mentioning is in regard to its pH value. Upon exploratory mixing with standardized cell media (containing a pH indicator) it was observed that the EMD might be slightly acidic, which may have a direct impact on cells. Upon this observation separate pH measurements were carried out on EMD which confirmed a pH slightly above 6 (data not shown). It is recommended to keep the pH in mind when applying EMD in cell-based experiments, since a suboptimal pH is known to negatively affect cell survival.

After initial assessments of EMD characteristics, it was decided to carry out 4 parallel modes of testing the effects of EMD upon HOrF. Microscopic observation and assessment of viable cell numbers constituted the main assessment of biological effects across these modes of testing.

### The 4 modes of testing were as follows:

A. Using EMD to pre-coat the plasticware onto which HOrF cells were to be seeded
B. Directly applying 100% EMD onto adherent HOrF cells for 5 minutes
C. Carrying out sub cultivation of HOrF cells with a 4:1 ratio of cell media and EMD supernatant
D. Carrying out sub cultivation of HOrF cells with a 4:1 ratio of cell media and EMD

For additional procedural details please see relevant project day reports.

### Results:

### A. Using EMD to pre-coat the plasticware onto which HOrF cells were to be seeded

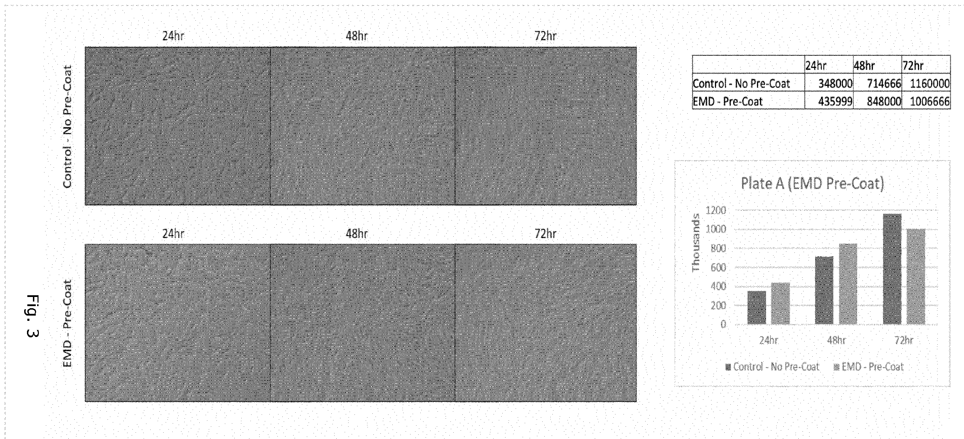

Assessment of cell numbers in wells analysed at 24hr, 48hr, and 72hr indicated nominal morphology and cell growth as compared to the control setting (see figure 3). There are no indications that EMD pre-coating confers any negative effect on the behaviour of HOrF cells in this mode of testing.

### B . Directly applying 100% EMD onto adherent HOrF cells for 5 minutes

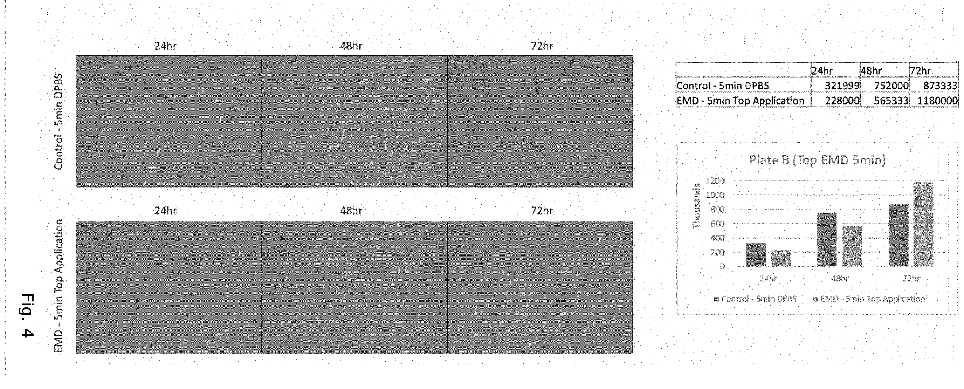

Assessment of cell numbers in wells analysed at 24hr, 48hr, and 72hr indicated temporary decrease in available cell numbers after EMD exposure, but with the cell numbers having normalized at the 72hr time-point (see figure 4). It was noted that the cells in the control setting for these samples showed signs of stress during the incubation (likely due to temporary serum withdrawal when they were kept in pure DPBS) which may have perturbed the growth curve. Please refer to the control condition of test mode A for a better appreciation of nominal control cell growth. Regarding the temporary disturbance in growth regarding the cells becoming exposed to EMD, one possible explanation may be that the undiluted EMD exposed the cells to a overly acidic environment which may have stunted the growth.

### C . Carrying out subcultivation of HOrF cells with a 4:1 ratio of cell media and EMD supernatant

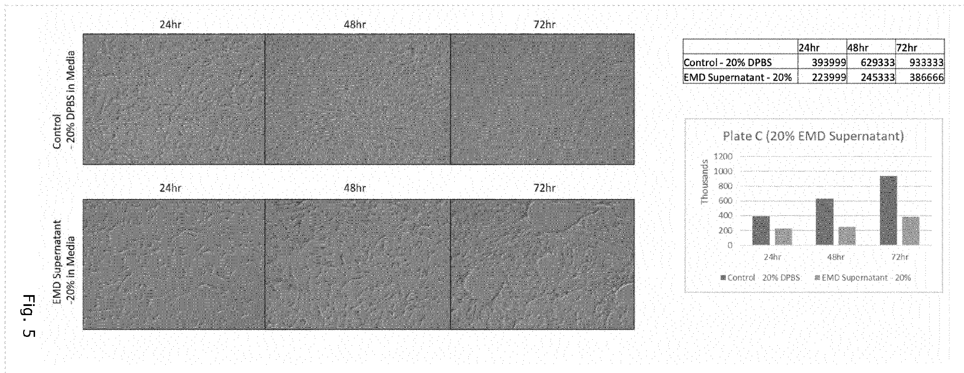

Assessment of cell numbers in wells analyzed at 24hr, 48hr, and 72hr indicated a clear disadvantage to cell growth in the wells that contained media with 20% EMD supernatant (see figure 5). It was observed that the HOrF cells, while slow, still managed to consistently expand during the duration of the experiment, indicating that EMD does not confer any acute toxic effects. The mechanism behind the slowed growth may potentially be linked to mechanical stress conferred to the cells by the particulate matter found in the EMD supernatant.

### D . Carrying out subcultivation of HOrF cells with a 4:1 ratio of cell media and EMD

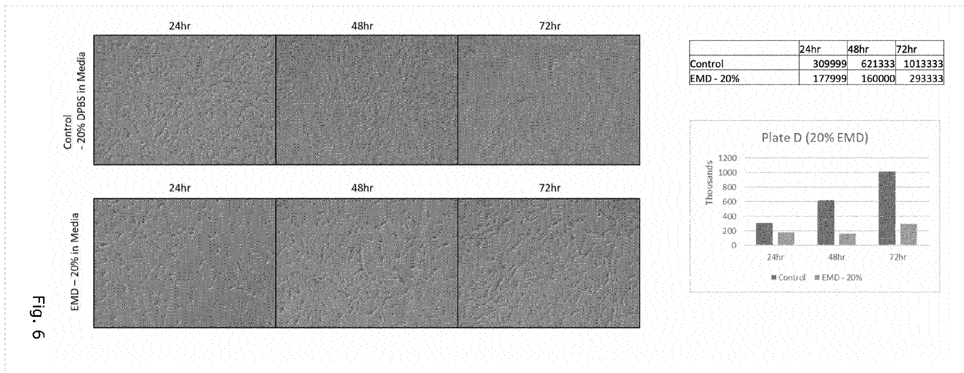

Assessment of cell numbers in wells analyzed at 24hr, 48hr, and 72hr indicated a significant disadvantage to cell growth in the wells that contained media with 20% EMD (see figure 6). It was observed that the HOrF cells, to a greater extent than in test mode C (EMD supernatant), had stunted growth during the duration of the experiment. The increased severity to cell growth demonstrated by the non-fractionated EMD (as compared to the EMD supernatant) further suggest that the negative effects seen may potentially be linked to mechanical stress conferred to the cells by the particulate matter found in the EMD supernatant (with the unfractionated EMD still containing the larger pieces of gel-like matter previously observed by microscopy). Since the pH is expected to be identical between unfractionated EMD and EMD supernatant, it is unlikely that the differential effect on cellular growth observed between test mode C and D can be explained by this parameter.

### Feasibility prediction regarding co-injection of EMD and HOrF into tissue

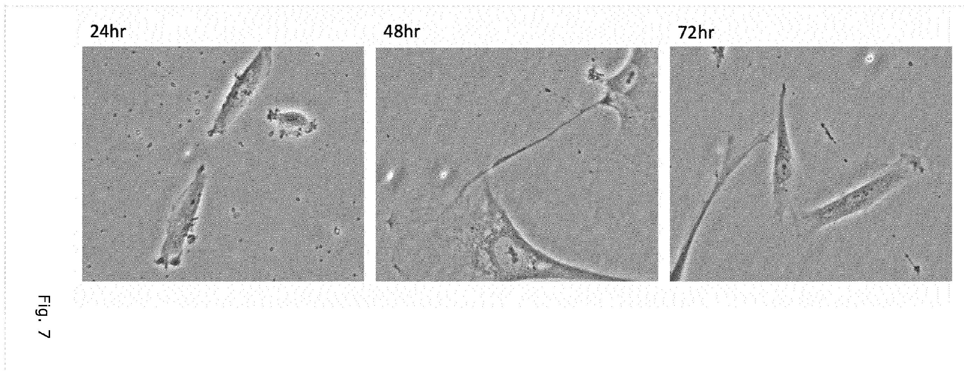

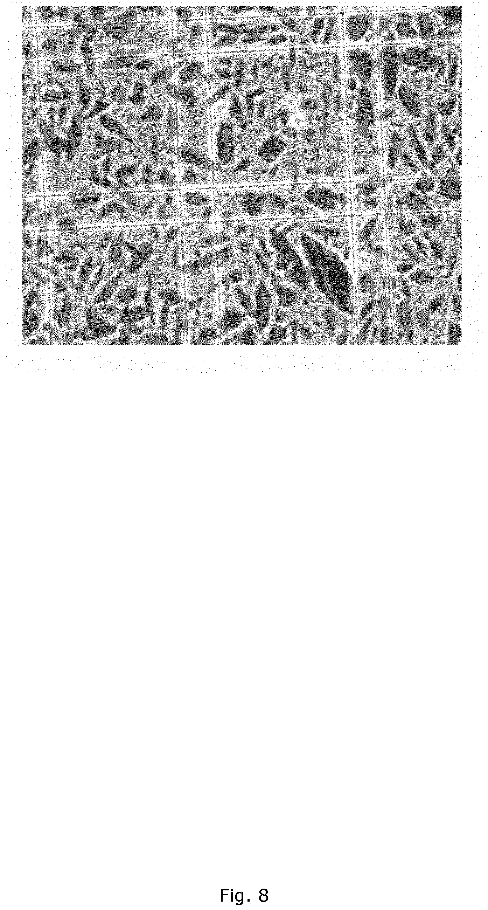

In addition to the tests described above, it was also of interest to evaluate if HOrF may survive EMD exposure when also being handled in a syringe (a potential prerequisite for in vivo transplantation). For this purpose, a suspension of HOrF cells in cell media were gently mixed at 1:1 with EMD while loaded into a syringe fitted with a 18G needle (see project day reports for procedural details). The HOrF / EMD suspension were then expunged into a well on a 6-well plate and allowed to incubate for 24hr, after which the well was washed and supplemented with fresh cell media. Subsequent observation indicated the presence of surviving cells in this well (see figure 7) although not in great numbers. One potential reason behind the low cell number may potentially be due to the particles of EMD blocking / preventing the majority of HOrF cells from successfully reaching and adhering to the bottom surface of the well (which is a prerequisite for HOrF cell survival). Nevertheless, the observation of surviving cells demonstrates that HOrF indeed can survive EMD exposure and handling in a syringe. In addition, using Trypan Blue staining on a suspension of cells in EMD (post centrifugation) it was confirmed that HOrF cells exposed to EMD retain viability (see figure 8) further indicating that EMD exposure is not acutely toxic to HOrF cells. However, due to the characteristics of EMD this test also demonstrate that centrifugation is not an efficient method for separating cells in suspension from the EMD.

### Discussion

In accordance with the original aims of this project efforts were made to provide data on EMD, its effect on HOrF, and it's potential to be administered through a syringe. Cell survival upon EMD exposure was confirmed, and it was demonstrated that both EMD and viable cells may be handled in a syringe (which is a prerequisite for injection into an in vivo environment). In addition, all expansion and stock production of HOrF were successfully carried out.

Composed as a heterogenous mixture of extracellular components, EMD have been proposed as a substrate capable of conferring distinct effects on cells, including a putative role in stimulating the formation of blood vessels (angiogenesis) as well as modulating inflammatory signalling. For better understanding how EMD may potentially be of value in a clinical setting it is of interest to better understand the effects of administering EMD into an in vivo tissue environment.

A clear pre-requisite for putative administration of EMD into tissue is confirming that it confers no acute negative effect on cellular viability. In this project, exposing HOrF cells to pure EMD for a set period of time was evaluated, and it was confirmed that exposed HOrF cells both retain viability as well as the capacity to proliferate. Interestingly, continuous EMD exposure on HOrF were found to limit the growth of these cells, but it is believed that cellular stress, originating from direct physical contact between the cells and the heterogeneous mixture of extracellular matrix material of the EMD suspension, is likely to be responsible for this effect, further supported by results that indicate a less severe effect when using a post centrifugation supernatant fraction of EMD (constituted of more homogenous and smaller extracellular matrix components). In regard to the in vitro experimental setup used in this project, it should be noted that the parameters of testing used differs significantly from the experimental parameters associated with putative EMD administration into an in vivo environment. Importantly, it should be noted that viable HOrF were confirmed to be present across all tested modes of EMD applications carried out in this project.

As demonstrated in this project, EMD is characterized as a heterogeneous mixture of extracellular matrix components, and that a more homogenous solution of such extracellular matrix components may be achieved if using a step of centrifugation. Furthermore, it was observed that EMD itself may have a different pH value than what may be ideal during cell cultivation. Thus, for the purpose of avoiding potential variability that may originate from individual preparations of EMD, it may be wise to consider using a combination of centrifugation and pH adjustment to help preparing more uniform EMD solutions.

### List of References

**1.** Gurtner GC, Werner S, Barrandon Y, Longaker MT. 2008. Wound repair and regeneration. Nature. 453(7193):314-321.
**2.** Greiling D, Clark RA. 1997. Fibronectin provides a conduit for fibroblast transmigration from collagenous stroma into fibrin clot provisional matrix. J Cell Sci. 110(Pt 7):861-870.
**3.** Yasuo Yamakoshi, et.al., Methods Mol Biol. 2019; 1922: 239-250.
**4.** US2013/0210735
**5.** Smith PC, Cáceres M, Martinez C, Oyarzún A, Martinez J. Gingival wound healing: an essential response disturbed by aging? J Dent Res. 2015 Mar;94(3):395-402. doi: 10.1177/0022034514563750. Epub 2014 Dec 19. PMID: 25527254; PMCID: PMC4814024.
6. Abraham S. at al, 2013 The Saudi Journal for Dental Research
7. Hammarström et al, 1997, J. Clin Periodontol; 24: 669-677.

### Items to which the invention relates are:

1. A pharmaceutical, dental and/or cosmetic composition comprising:
   a. a cell culture population of human fibroblasts (hPFB) and/or human periodontal ligament cells (hPDL), and
   b. purified and/or isolated enamel matrix derivative (EMD) proteins, which have a molecular weight between 1-160kDa.
2. A pharmaceutic dental and/or cosmetic composition according to claim 1, further comprising:
   c. a suitable pharmaceutical carrier.
3. A pharmaceutic dental and/or cosmetic composition according to claim 1 or 2, wherein the cells have been lyophilized before being added to the composition.
4. A pharmaceutic dental and/or cosmetic composition according to any of the preceding claims, wherein the EMD proteins have been lyophilized before being added to the composition.
5. A pharmaceutic dental and/or cosmetic composition according to any of the preceding claims, wherein the cells are oral human fibroblasts (hPFB).
6. A pharmaceutic dental and/or cosmetic composition according to any of the preceding claims, wherein the EMD proteins are isolated porcine Enamel Matrix Derivative (EMD) proteins.
7. A pharmaceutic dental and/or cosmetic composition according to any of the preceding claims, wherein the carrier has a neutral pH.
8. A pharmaceutic dental and/or cosmetic composition according to any of the preceding claims, wherein the carrier has a pH between 7-7.75.
9. A pharmaceutic dental and/or cosmetic composition according to any of the preceding claims, which is a solution, a liquid and/or an aqueous fluid.
10. A pharmaceutic dental and/or cosmetic composition according to any of the preceding claims, wherein the carrier is EDTA or an alginate carrier.
11. A pharmaceutic dental and/or cosmetic composition according to any of the preceding claims, wherein the carrier is Propylene Glycol Alginate (PGA).
12. A pharmaceutic dental and/or cosmetic composition according to any of the preceding claims, which is in the form of an injectable gel.
13. A pharmaceutic dental and/or cosmetic composition according to any of the preceding claims, which is a lyophilized powder.
14. A pharmaceutic dental and/or cosmetic composition according to any of the preceding claims, wherein the EMD proteins comprise amelogenins, enamelin, tuft protein, proteases and albumin.
15. A pharmaceutic dental and/or cosmetic composition according to any of the preceding claims, wherein at least 80% of the EMD proteins are amelogenins.
16. A pharmaceutic dental and/or cosmetic composition according to any of the preceding claims, which further comprises one or more growth factor(s).
17. A pharmaceutic dental and/or cosmetic composition according to any of the preceding claims, for use in medicine.
18. A pharmaceutic dental and/or cosmetic composition according to any of the preceding claims, for use in minimally-invasive surgery.
19. A pharmaceutic dental and/or cosmetic composition according to any of the preceding claims, which is administered by injection.
20. A pharmaceutic dental and/or cosmetic composition according to any of the preceding claims, which is administered by injection into soft tissue in the oral cavity and/or the craniomaxillofacial complex (CMF).
21. A pharmaceutic dental and/or cosmetic composition according to any of the preceding claims, for use in inducing and/or promoting novel formation of soft tissue in the oral cavity and/or the craniomaxillofacial complex (CMF).
22. A pharmaceutic dental and/or cosmetic composition according to any of the preceding claims, for use in promoting growth and/or healing of soft tissue in the oral cavity and/or the craniomaxillofacial complex (CMF).
23. A pharmaceutic dental and/or cosmetic composition according to any of the preceding claims, for use in promoting reinforcement and/or thickening of gingiva in patients with a thin gingiva biotype.
24. A pharmaceutic dental and/or cosmetic composition according to any of the preceding claims, for use in treating a gingival deficiency and/or disorder selected from the group consisting of gingival thin biotype, gingival recession and enlargement and gingival papilla regeneration.
25. A pharmaceutic dental and/or cosmetic composition according to any of the preceding claims, for use in promoting gingival sealing around dental implants (periimplantitis prevention and treatment) and/or alveolar ridge preservation.
26. A pharmaceutic dental and/or cosmetic composition according to any of the preceding claims, for use in extraction socket management, such as for treating intrabony pockets and/or promoting alveolar ridge regeneration (periodontology)
27. A pharmaceutic dental and/or cosmetic composition according to any of the preceding claims, for use in combination with an implantable 3D scaffold, such as a 3D scaffold-sponge.
28. A pharmaceutic dental and/or cosmetic composition according to any of the preceding claims, for use in treating a deficiency and/or disorder selected from the group consisting of gingival recession and/or enlargement, alveolar ridge augmentation (implant dentistry), and intrabony pockets and/or alveolar ridge regeneration (periodontology), and/or for promoting alveolar ridge preservation and/or extraction socket management.
29. A kit for comprising:
   a. a lyophilized cell culture population of human fibroblasts (hPFB) and/or human periodontal ligament cells (hPDL), and
   b. lyophilized purified and/or isolated enamel matrix derivative (EMD) proteins, which have a molecular weight between 1-160kDa.
30. A kit according to claim 29, further comprising
   c. a suitable pharmaceutical carrier.
31. A kit according to claim 29 or 30, wherein the cells are oral human fibroblasts (hPFB).
32. A kit according to any one of claims 29-31, wherein the EMD proteins are isolated porcine Enamel Matrix Derivative (EMD) proteins.
33. A kit according to any one of claims 29-32, wherein the carrier has a neutral pH.
34. A kit according to any one of claims 29-33, wherein the carrier has a pH between 7-7.75.
35. A kit according to any one of claims 29-34, for producing/manufacturing a pharmaceutic dental and/or cosmetic composition in in the form of a powder, an injectable gel, an injectable solution, a liquid and/or an aqueous fluid, for use according to any one of claims 17-28.
36. A method of treating a patient suffering from a gingival deficiency and/or disorder selected from the group consisting of gingival thin biotype, gingival recession and enlargement and gingival papilla regeneration, wherein said patient is injected with a composition according to any one of claims 1-16.

## Claims

1. A pharmaceutical, dental and/or cosmetic composition comprising:
a. a cell culture population of human fibroblasts (hPFB) and/or human periodontal ligament cells (hPDL), and
b. purified and/or isolated enamel matrix derivative (EMD) proteins, which have a molecular weight between 1-160kDa.

2. A pharmaceutic dental and/or cosmetic composition according to claim 1, further comprising:
c. a suitable pharmaceutical carrier.

3. A pharmaceutic dental and/or cosmetic composition according to claim 1 or 2, wherein the cells have been lyophilized before being added to the composition.

4. A pharmaceutic dental and/or cosmetic composition according to any of the preceding claims, wherein the EMD proteins have been lyophilized before being added to the composition.

5. A pharmaceutic dental and/or cosmetic composition according to any of the preceding claims, wherein the cells are oral human fibroblasts (hPFB).

6. A pharmaceutic dental and/or cosmetic composition according to any of the preceding claims, wherein the EMD proteins are isolated porcine Enamel Matrix Derivative (EMD) proteins.

7. A pharmaceutic dental and/or cosmetic composition according to any of the preceding claims, which is in the form of an injectable gel.

8. A pharmaceutic dental and/or cosmetic composition according to any of the preceding claims, which is a lyophilized powder.

9. A pharmaceutic dental and/or cosmetic composition according to any of the preceding claims, for use in medicine.

10. A pharmaceutic dental and/or cosmetic composition according to any of the preceding claims, for use in minimally-invasive surgery.

11. A pharmaceutic dental and/or cosmetic composition according to any of the preceding claims, which is administered by injection.

12. A pharmaceutic dental and/or cosmetic composition according to any of the preceding claims, which is administered by injection into soft tissue in the oral cavity and/or the craniomaxillofacial complex (CMF).

13. A pharmaceutic dental and/or cosmetic composition according to any of the preceding claims, for use in inducing and/or promoting novel formation of soft tissue in the oral cavity and/or the craniomaxillofacial complex (CMF), for use in promoting growth and/or healing of soft tissue in the oral cavity and/or the craniomaxillofacial complex (CMF), for use in promoting reinforcement and/or thickening of gingiva in patients with a thin gingiva biotype, for use in treating a gingival deficiency and/or disorder selected from the group consisting of gingival thin biotype, gingival recession and enlargement and gingival papilla regeneration, for use in promoting gingival sealing around dental implants (periimplantitis prevention and treatment) and/or alveolar ridge preservation, for use in extraction socket management, such as for treating intrabony pockets and/or promoting alveolar ridge regeneration (periodontology), for use in combination with an implantable 3D scaffold, such as a 3D scaffold-sponge, or for use in treating a deficiency and/or disorder selected from the group consisting of gingival recession and/or enlargement, alveolar ridge augmentation (implant dentistry), and intrabony pockets and/or alveolar ridge regeneration (periodontology), and/or for promoting alveolar ridge preservation and/or extraction socket management.

14. A kit for comprising:
a. a lyophilized cell culture population of human fibroblasts (hPFB) and/or human periodontal ligament cells (hPDL), and
b. lyophilized purified and/or isolated enamel matrix derivative (EMD) proteins, which have a molecular weight between 1-160kDa.

15. A kit according to claim 14, further comprising
c. a suitable pharmaceutical carrier.
